# EUROPEAN PATENT SPECIFICATION

(11) **EP 1 862 804 B1**
(45) Date of publication and mention of the grant of the patent: **05.05.2010**
(21) Application number: 06715708.1
(22) Date of filing: 14.03.2006
(51) Int. Cl.: G01N 33/574, C07K 16/18, G01N 27/62

(54) **METHOD FOR DIAGNOSIS OF PROSTATE CANCER**
VERFAHREN ZUR DIAGNOSE VON PROSTATAKREBS
MÉTHODE POUR DIAGNOSTIQUER LE CANCER DE LA PROSTATE

(30) Priority: 14.03.2005 JP 2005071387; 23.08.2005 JP 2005241414
(43) Date of publication of application: 05.12.2007
(73) Proprietor: Link Genomics, Inc., Chuo-ku Tokyo 103-0024 (JP); OSAKA UNIVERSITY, Suita-shi, Osaka 565-0871 (JP)
(72) Inventor: TSUJIKAWA, KAZUTAKE, Suita-shi, Osaka 5650871 (JP); YAMAMOTO, Hiroshi, Suita-shi, Osaka 5650871 (JP); KONISHI, Noboru, Kashihara-shi, Nara 6348521 (JP)
(74) Representative: Baldock, Sharon Claire
(86) International application number: PCT/JP2006/305480
(87) International publication number: WO 2006/098464

(56) References cited:
- KONISHI N ET AL: "High expression of a new marker PCA-1 in human prostate carcinoma" CLINICAL CANCER RESEARCH, THE AMERICAN ASSOCIATION FOR CANCER RESEARCH, vol. 11, no. 14, 15 July 2005 (2005-07-15), pages 5090-5097, XP003003791 ISSN: 1078-0432
- KAN S. ET AL.: 'Zenritsusengan ni okeru Prostate Cancer Antigen-1 (PCA-1) no Hatsugen to Kino' NIPPON YAKUGAKUKAI NENKAI KOEN YOSHISHU vol. 124, no. 3, 2004, page 110, XP003005830
- TSUJIKAWA K. ET AL.: 'DNA, RNA Datsu Methylete Koso PCA-1 (hABH3) no Cloning to Hatsugen Kaiseki' NIPPON GAN GAKKAI SOKAI KIJI vol. 62, 2003, pages 387 - 388, XP003005831

## Description

### TECHNICAL FIELD

The present invention relates to a method for diagnosing prostate cancer, and specifically to a method for diagnosing prostate cancer using PCA-1 in a body fluid sample (e.g., blood, urine) of a subject as a marker, and a kit therefor.

### BACKGROUND ART

Prostate cancer occurs to a large number of people and causes about 20% of deaths of male cancer patients in the European and North American countries. In Japan also, the number of people having prostate cancer is increasing as more people take Western style cuisine and live to an older age. In Japan, the number of prostate cancer patients is largest among the number of patients of cancers of the urinary area. It is of a great medical significance to diagnose prostate cancer at an early stage.

Currently, prostate-specific antigen (PSA) is generally used to diagnose prostate cancer, and many kits for detecting PSA in blood components using an antigen-antibody reaction are commercially available. Blood PSA measurement is advantageous in being capable of detecting the presence of prostate cancer sensitively, and also in being capable of estimating the stage of the disease because the PSA value is increased as the cancer progresses.

However, the blood PSA measurement has problems that the blood PSA value is often increased by prostatomegaly or prostatitis, and that in an early stage of prostate cancer, the PSA value may be occasionally normal and thus a false negative result is provided. Thus, in order to make a definite diagnosis as prostate cancer, tissue diagnosis is required in which a tissue of prostate is sampled by fine needle aspiration biopsy, or transrectal or transperineal needle biopsy, and examined with a microscope.

On the other hand, there is almost no knowledge for estimating which protein is antigenic or increases its concentration in biological fluids, among the proteins that are hyperexpressed in cancer. There are a great number of genes and proteins which are hyperexpressed in prostate cancer cells or tissues, but all these many amplified genes or proteins are not necessarily usable as a diagnosis marker of prostate cancer. For example, many cDNAs expressed in prostate cancer are currently commercially available from Invitrogen, but the kinetics of these genes in a prostate cancer patient are totally unknown. There is a report that PCA-1 (prostate cancer antigen-1) has been identified as a gene which is hyperexpressed in prostate cancer cells or tissues (Proceedings of the 123rd Annual Meeting of the Pharmaceutical Society of Japan Vol. 4, page 15, 2003).

### DISCLOSURE OF THE INVENTION

Currently, diagnosis of prostate cancer imposes a heavy burden on patients and physicians and requires a great amount of work and time. In such a situation, a novel method for diagnosing prostate cancer more simply and rapidly and with fewer burdens on the patients and physicians is desired. Especially, a novel prostate cancer marker realizing rapid and reliable diagnosis using a body fluid (e.g., blood, urine) sample is desired to be developed.

In order to solve the above-described problems, the present invention provides a method, a kit and a diagnostic agent for detecting PCA-1, PCA-1 gene, PCA-1 mRNA or anti-PCA-1 autoantibody as a prostate cancer marker in a body fluid sample derived from a subject as described below.
(1) A method for diagnosing prostate cancer comprising detecting and/or quantifying PCA-1 in a body fluid sample derived from a subject as a prostate cancer marker.
(2) The method according to (1), wherein the body fluid sample is whole blood, serum, plasma or urine.
(3) The method according to (1) or (2), wherein the PCA-1 is detected and/or quantified using a mass spectrometer.
(4) The method according to (1) or (2), wherein the PCA-1 is detected and/or quantified using an anti-PCA-1 antibody.
(5) The method according to (4) comprising the steps of:
   bringing the body fluid sample into contact with the anti-PCA-1 antibody; and
   detecting and/or quantifying the binding between the PCA-1 and the anti-PCA-1 antibody in the body fluid sample.
(6) The method according to (5), wherein the step of detecting and/or quantifying comprises detecting and/or quantifying the binding between the PCA-1 and the anti-PCA-1 antibody using a labeled anti-PCA-1 antibody.
(7) The method according to any one of (4) through (6), which is performed in accordance with an immunoassay selected from the group consisting of Western blotting, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, and protein A immunoassay.
(8) Use of An anti-PCA-1 antibody as a prostate cancer diagnostic agent on a body fluid sample.
(9) Use of A polynucleotide formed of a nucleotide sequence hybridizable with the nucleotide sequence of the PCA-1 gene, or PCA-1 mRNA under stringent hybridization conditions as a prostate cancer diagnostic agent on a body fluid sample.
(10) The use according to claim 8 or claim 9 for use on a body fluid sample, wherein the body fluid sample is whole blood, serum plasma or urine.
(11) Use of a kit for the diagnosis of prostate cancer, wherein the use is on a body fluid sample, the kit comprising an anti-PCA-1 antibody.
(12) Use according to (11), the kit further comprising a labeled anti-PCA-1 antibody.
(13) Use according to (11) or (12), wherein detection and/or quantification of PCA-1 is performed in accordance with an immunoassay selected from the group consisting of Western blotting, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, and protein A immunoassay.
(14) Use of a kit for the diagnosis of prostate cancer, wherein the use is on a body fluid sample, the kit comprising a polynucleotide formed of a nucleotide sequence hybridizable with the nucleotide sequence of the PCA-1 gene, or PCA-1 mRNA under stringent hybridization conditions.
(15) Use of a kit according to any one of (11) through (14), wherein the body fluid sample is whole blood, serum, plasma, or urine.
(16) A method for diagnosing prostate cancer comprising detecting and/or quantifying an anti-PCA-1 autoantibody in a body fluid sample derived from a subject as a prostate cancer marker.
(17) The method according to (16), wherein the body fluid sample is whole blood, serum or plasma.
(18) The method according to any one of (16) or (17), wherein the anti-PCA-1 autoantibody is detected and/or quantified using a PCA-1 antigen.
(19) The method according to (18), comprising the steps of:
   bringing the body fluid sample into contact with the PCA-1 antigen; and
   detecting and/or quantifying the binding between the anti-PCA-1 autoantibody and the PCA-1 antigen in the body fluid sample.
(20) The method according to (19), wherein the step of detecting and/or quantifying comprises detecting and/or quantifying the binding between the PCA-1 and the anti-PCA-1 autoantibody using a labeled antibody against the anti-PCA-1 autoantibody.
(21) The method according to any one of (16) through (20), which is performed in accordance with an immunoassay selected from the group consisting of Western blotting, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, and protein A immunoassay.
(22) A kit for detecting and/or quantifying an anti-PCA-1 autoantibody in a body fluid sample derived from a subject as a prostate cancer marker, the kit comprising a PCA-1 antigen.
(23) The kit according to (22)further comprising a labeled antibody against the anti-PCA-1 autoantibody so as to use the labeled antibody for detecting and/or quantifying the binding between the PCA-1 antigen and the anti-PCA-1 autoantibody.
(24) Use according to (22) or (23), wherein detection and/or quantification of an anti-PCA-1 antibody is performed in accordance with an immunoassay selected from the group consisting of Western blotting, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, and protein A immunoassay.

Also described is a prostate cancer diagnostic agent for detecting and/or quantifying an anti-PCA-1 autoantibody in a body fluid sample derived from a subject as a prostate cancer market, the agent comprising a PCA-1 antigen.

The present invention provides a method using PCA-1 as a prostate cancer market in a body fluid (e.g., blood, urine) sample from a subject, for the first time in history. The present invention can provide an accurate prostate cancer diagnostic method capable of diagnosing prostate cancer by a single examination. This method, when combined with an existing prostate cancer diagnostic method as necessary, realizes more reliable prostate cancer diagnosis. The present invention provides a rapid and simple prostate cancer diagnostic method which can be used for performing noninvasive or low-invasion measurement using a body fluid sample and thus imposes fewer burdens both on patients and physicians. The present invention is useful for diagnosis of prostate cancer and also for catamnestic evaluation on cancer, identification of a subject having predisposition of cancer, and observation of a patient under cancer treatment.

### BRIEF DESCRIPTION OF THE DRAWINGS

Fig. 1 shows an elution profile of a blood sample derived from a subject having prostate cancer, which was monitored by an LC-MS mass spectrometer.

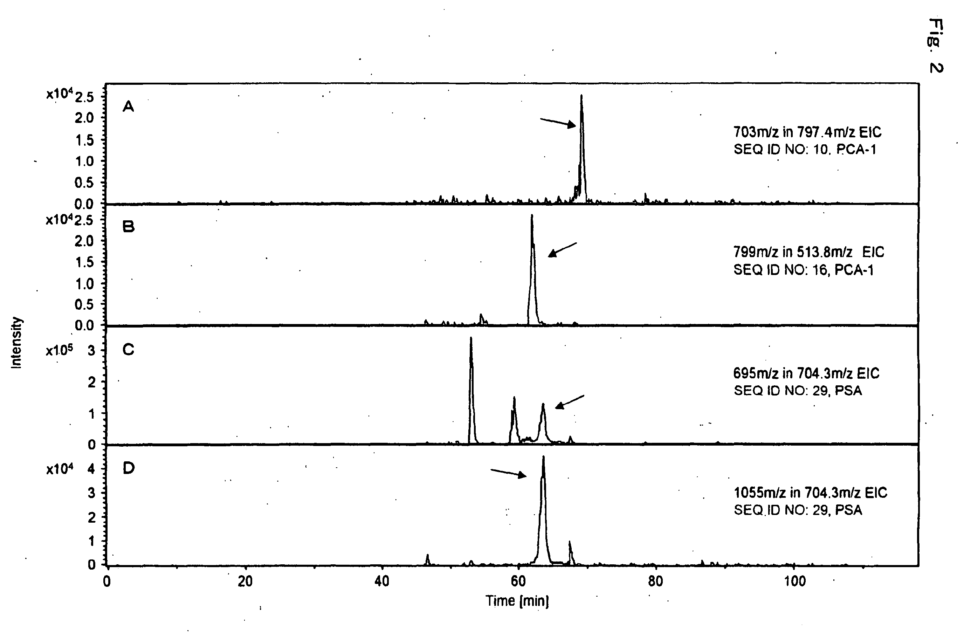

Fig. 2 shows, in parts A and B, elution profiles when molecules having a mass of a triypsin-degraded peptide fragment of PCA-1 obtained by an *in silico* calculation or a mass of cleaved peptides which can be generated from the peptide fragment are selectively monitored by an LC-MS mass spectrometer; and also shows, in parts C and D, elution profiles when molecules having a mass of a triypsin-degraded peptide fragment of PSA obtained by an *in silico* calculation or a mass of cleaved peptides which can be generated from the peptide fragment are selectively monitored by the LC-MS mass spectrometer for comparison.
Fig. 3 shows an amino acid sequence determined by MSMS analysis performed on the PCA-1 peak observed around the retention time of 68 minutes as shown in Fig. 2, part A.
Fig. 4 shows an amino acid sequence determined by MSMS analysis performed on the PCA-1 peak observed around the retention time of 62 minutes as shown in Fig. 2, part B.
Fig. 5 shows, for comparison, an amino acid sequence determined by MSMS analysis performed on the PSA peak observed around the retention time of 64 minutes as shown in Fig. 2, parts C and D.
Fig. 6 shows, as a comparative example to the experiment shown in Fig. 2, elution profiles of a blood sample derived from a healthy subject, which were pre-treated and trypsin-treated, the elution profiles being obtained when molecules having a specific mass are monitored in the same measurement conditions as in the experiment shown in Fig. 2.
Fig. 7 shows a photograph illustrating the results of Western blotting analysis, which indicate the presence of PCA-1 in urine samples from prostate cancer patients.

### BEST MODE FOR CARRYING OUT THE INVENTION

The present inventors developed a novel technique using a mass spectrometer for rapidly analyzing the presence, and a quantity of, a target polypeptide in the blood, and attempted to detect PCA-1 in a blood sample using the technique. As a result, the present inventors confirmed that PCA-1 is present in a blood sample derived from a prostate cancer patient and that PCA-1 is not substantially detected in a blood sample derived from a healthy subject. The present invention is based on such novel knowledge, and demonstrates that PCA-1 is usable as a prostate cancer marker in a body fluid sample such as blood, for the first time in history.

The present inventors also confirmed that PCA-1 is detected in a urine sample from a prostate cancer patient and that PCA-1 is detected only in trace amounts or is not substantially detected in a urine sample from a healthy subject. The present invention also demonstrates that PCA-1 is usable as a prostate cancer marker in a body fluid sample such as urine, for the first time in history.

Accordingly, in one embodiment, the present invention provides a method for detecting and/or quantifying PCA-1 in a body fluid sample derived from a subject as a prostate cancer marker. The method according to the present invention is applicable to, for example, diagnosis or catamnestic evaluation of prostate cancer.

In another embodiment, the present invention provides a method for detecting and/or quantifying an anti-PCA-1 autoantibody in a body fluid sample derived from a subject as a prostate cancer marker. Namely, the present invention provides a method for detecting and/or quantifying the expression of an anti-PCA-1 autoantibody in a body fluid, the method being applicable to, for example, diagnosis or recuperation evaluation of prostate cancer.

### (Detection and/or quantification of PCA-1 or an anti-PCA-1 autoantibody in a body fluid)

There are many methods for detecting the PCA-1 protein in a body fluid sample such as blood or urine taken from a subject. Any of these methods is usable for the detection. Other than a method using a mass spectrometer as described above, an immunoassay for detecting the PCA-1 protein with an antibody specifically bindable to the PCA-1 protein (anti-PCA-1 antibody) is usable, for example. An antibody useful for the present invention is usable for quantitatively or qualitatively detecting the presence of the PCA-1 protein or a fragment thereof. Accordingly, in one embodiment, the present invention provides a method for detecting a prostate cancer marker in a body fluid sample derived from a subject, using an anti-PCA-1 antibody.

There are many methods for detecting a PCA-1 polynucleotide in a body fluid sample such as blood or urine taken from a subject. Any of these methods is usable for the detection. Examples of the detectable polynucleotide include recombinant DNA and RNA molecules including PCA-1 gene or a fragment thereof, PCA-1 mRNA, selective splice variant type PCA-1 mRNA, and PCA-1 polynucleotide.

There are many methods well known in the art for amplifying a PCA-1 polynucleotide and/or detecting the presence thereof, which are usable to carry out this aspect of the present invention. For example, in one embodiment of the present invention, a method for detecting PCA-1 mRNA in a body fluid sample comprises the steps of: producing cDNA from a sample by reverse transcription using at least one primer, amplifying the cDNA thus produced using a PCA-1 polynucleotide as sense and antisense primers in order to amplify PCA-1 cDNA in the cDNA, and detecting the presence of the amplified PCA-1 cDNA. In another embodiment of the present invention, a method for detecting a PCA-1 gene in a body fluid sample comprises the steps of: first isolating genomic DNA from the body fluid sample, amplifying the isolated genomic DNA using a PCA-1 polynucleotide as sense and antisense primers in order to amplify a PCA-1 gene in the genomic DNA, and detecting the presence of the amplified PCA-1 gene.

As used herein, the terms "PCA-1" and "PCA-1 protein" each refers to the human PCA-1 protein, the gene of which is specified as GenBank Accession No. AB042029. The amino acid sequence of the PCA-1 protein is specified as Accession No. NP_631917 (SEQ ID NO: 2) of the publicly accessible protein database. As used herein, the term "PCA-1" used in connection with an embodiment in which PCA-1 in a body fluid sample is utilized as a prostate cancer marker encompasses the full length PCA-1 protein as well as protease-degraded products of PCA-1, fragments of PCA-1, and derivatives thereof Herein, the term "derivative" encompasses a peptide or a polypeptide containing mutations, substitutions, deletions and/or additions of 1 or several (e.g., six) amino acid residues in the amino acid sequence of the PCA-1 protein or a fragment thereof, and having substantially the same antigen specificity as the PCA-1 protein. Typical examples of the derivative include PCA-1 polymorphism, sequence change by splicing and the like.

As used herein when referring to an embodiment in which PCA-1 in a body fluid sample is used as a prostate cancer marker, the term "anti-PCA-1 antibody" encompasses antibodies specifically bindable to PCA-1, protease-degraded products of PCA-1, fragments of PCA-1, or derivatives thereof The protease-degraded products or fragments may have any length with no specific limitation as long as being recognizable as an antigen specific to the anti-PCA-1 antibody. However, the length is preferably at least 6 amino acids, more preferably at least 8 amino acids, and still more preferably at least 10 amino acids (for example, the amino acid sequence: RRAPEPRVIDREG (SEQ ID NO: 34) or a fragment containing a partial sequence thereof). These degradation products or fragments may be an arbitrary part of the PCA-1 protein, but preferably correspond to an epitope of the PCA-1 protein or contain a part corresponding to the epitope. The term "derivative" encompasses a peptide or a polypeptide containing mutations, substitutions, deletions and/or additions of 1 or several (e.g., six) amino acid residues in the amino acid sequence of the PCA-1 protein, a protease-degraded product thereof, or a fragment thereof, and having substantially the same antigen specificity as the PCA-1 protein.

As used herein, the terms "PCA-1", "PCA-1 protein" and "PCA-1 antigen" used in connection with an embodiment in which an anti-PCA-1 autoantibody in a body fluid sample is used as a prostate cancer marker encompass the PCA-1 protein as well as fragments containing an epitope of the PCA-1 protein or being recognizable as the epitope by the anti-PCA-1 antibody, and derivatives thereof Such fragments may have any length with no specific limitation as long as being recognizable as an antigen specific to the anti-PCA-1 antibody, but the length is preferably at least 6 amino acids, more preferably at least 8 amino acids, and still more preferably at least 10 amino acids. These degradation products or fragments may be an arbitrary part of the PCA-1 protein, but preferably correspond to an epitope of the PCA-1 protein or contain a part corresponding to the epitope. The "derivative" encompasses a peptide or a polypeptide containing mutations, substitutions, deletions and/or additions of 1 or several (e.g., six) amino acid residues in the amino acid sequence of the PCA-1 protein, or a fragment thereof, and having substantially the same antigen specificity as the PCA-1 protein.

As used herein, when referring to an antibody which "specifically binds" to a protein, a degradation product thereof or a fragment thereof, it means that the antibody binds to a specific amino acid sequence of such a protein, degradation product or fragment with substantially higher affinity than to other amino acid sequences. Herein, the expression "substantially higher affinity" means a sufficiently high level of affinity at which the specific amino acid sequence is detectable as being distinguished from other amino acid sequences by a desired measuring device.

As used herein, the term "prostate cancer marker" refers to a molecule in a body fluid (e.g., blood, urine, lymph fluid, saliva, perspiration, semen, etc.), a cell or a tissue of a subject, which is not derived from a normal prostate tissue or which is selectively hyperexpressed in a prostate cancer cell or tissue. The presence of such a molecule in a body fluid, cell or tissue of a subject indicates or suggests the presence of prostate cancer.

As used herein, the term "body fluid sample" encompasses a body fluid sample such as blood, urine, lymph fluid, saliva, perspiration, semen or the like derived from a subject. Preferable examples of the body fluid sample used in the present invention are blood and urine. A blood sample is especially preferable.

As used herein, the term "blood sample" refers to a blood sample obtained from a subject. The blood sample is not limited to whole blood and encompasses blood component fractions such as serum, plasma and the like, as well as blood component fractions, blood products and the like from which albumin, immunoglobulin and the like are removed.

As used herein, the term "prostate cancer" represents the concept widely encompassing cancer generated in prostate, and encompasses adenocarcinoma as well as squamous cell carcinoma, transitional cell carcinoma, neuroendocrine carcinoma, undifferentiated carcinoma and the like, which are generated in prostate. Preferably, the prostate cancer is adenocarcinoma generated in prostate.

As used herein, the term "subject" generally refers to a human from who a body fluid sample is taken to be examined using a method according to the present invention, but mainly refers to human male. More specifically, the term "subject" encompasses, for example, human male to be examined for a medical checkup, and human male suspected to have prostate cancer because of subjective symptoms (e.g., sensation of residual urine), result of palpation (lump in prostate, etc.), examination result of PSA concentration in serum, etc. (high value of PSA concentration, etc.) or the like.

### (Antibody)

The anti-PCA-1 antibody used in the present invention may be a polyclonal antibody or a monoclonal antibody. The term "antibody" generally encompasses an arbitrary antibody fragment or derivative of the monoclonal or the polyclonal antibody, especially a fragment or derivative having substantially the same antigen specificity as the monoclonal or the polyclonal antibody. The latter encompasses antibody fragment (Fab, Fab'₂, CDR, etc.), humanized antibody, polyfunctional antibody, single-chain antibody (ScFv) and the like. The antibody used in the present invention can be produced by conventional methods, for example, by immunizing an animal and recovering serum (polyclonal) or spleen cells (for producing a hybridoma through fusion with an appropriate cell system).

The antibody is not limited to any specific class. An antibody having any isotype such as IgG; IgM, IgA, IgD, IgE or the like is usable. Preferably, the antibody is IgG or IgM. In consideration of ease of purification or the like, IgG is more preferable.

A method for producing a polyclonal antibody derived from various species including mouse, rodent, primate, equine, swine, rabbit, fowl and the like is found in, for example, Vaitukaitis et al. (Vaitukaitis, Robbins et al., J Clin Endocrinol Metab. 33(6): 988-91, 1971). An antigen is combined with an adjuvant (e.g., Freund's adjuvant) and administered to an animal, typically by subcutaneous injection. The injection may be performed in repetition. The blood sample is recovered and immunoglobulin or serum is isolated.

A method for producing a monoclonal antibody from different species is found in, for example, Harlow et al. (Harlow and Lane (ed.), Antibodies: a laboratory manual, Cold Spring Harbor Laboratory, Cold Spring Harbor, N.Y., pp. 139-282. 1988) or Kohler et al. (Kohler and Milstein, Nature 256 (5517): 495-7, 1975). This method comprises immunizing an animal with an antigen, recovering spleen cells, and then fusing them with immortalized cells such as myeloma cells or the like. The obtained hybridoma generates a monoclonal antibody, and individual clones can be isolated by selection with limiting dilution. The antibody can be also produced by selection of combinatorial immunoglobulin library as disclosed in, for example, Ward et al. (Ward, Gussow et al., Nature 341(6242): 544-6, 1989).

The Fab or Fab'₂ fragment can be produced by a conventional digestion method using protease (e.g., pepsin or papain). The humanized antibody can be prepared by one of the methods described in, for example, Riechmann et al. (Riechmann, J Mol Biol., Oct 5; 203(3): 825-8, 1988), and Jones et al. (Jones et al., Nature 321: 522-525, 1986).

The chimera antibody can be produced with reference to, for example, "Jikken Igaku (Special Supplement Issue), Vol. 1.6, No.10, 1988", Japanese Patent Publication for Opposition No. 3-73280 and the like. The humanized antibody can be produced with reference to, for example, "Nature Genetics,Vol. 15, pp. 146-156, 1997", "Nature Genetics, Vol. 7, pp. 13-21, 1994", PCT National Phase Japanese Laid-Open Patent Publication No. 4-504365, WO94/25585, "Nikkei Science, June issue, pp. 40-50, 1995", "Nature, Vol. 368, pp. 856-859, 1994", PCT National Phase Japanese Laid-Open Patent Publication No. 6-500233 and the like.

### (Immunoassay)

### 1. Assay for detecting PCA-1

In one embodiment of immunoassay useful for carrying out the present invention, a body fluid sample derived from a subject and an anti-PCA-1 antibody are brought into contact with each other, and then an immunocomplex of the PCA-1 and the anti-PCA-1 antibody in the body fluid sample is detected.

The immunoassay in the above-described embodiment according to the present invention can use a measuring system using the Western blotting technique as well as, for example, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, protein A immunoassay and the like. With such techniques, the anti-PCA-1 antibody is usually labeled with, for example, fluorescent group, luminescent group, free radical group, particle, bacteriophage, cell, metal, enzyme, complement enzyme, radioisotope or the like.

In addition, agents other than antibody, such as a polypeptide specifically binding to the PCA-1 protein and the like, are usable for measuring the expression level of the PCA-1 protein.

The immunoassay for detecting the expression of PCA-1 typically comprises bringing a body fluid sample taken from a subject suspected of having prostate cancer or a subject with a risk of having prostate cancer into contact with an anti-PCA-1 antibody under conditions that allow a specific antigen-antibody binding and then measuring the amount of specific immunobinding of the antibody. In a specific embodiment, for example, such antibody binding is usable to detect presence and/or amplified expression of the PCA-1 protein. In this case, detection of an amplified expression of the PCA-1 protein is an indicator of the state of disease. When necessary, the level of the PCA-1 protein in the body fluid sample may be compared with the level in the sample from a healthy subject with no prostate cancer.

In one embodiment of the immunoassay described above, a body fluid sample such as a serum sample or the like is put into contact with a solid phase support or carrier such as nitrocellulose, for the purpose of immobilizing all the proteins that are present in the sample. Next, this support is washed with a buffer solution and then treated with an anti-PCA-1 antibody detectably labeled. Then, this solid phase support is washed twice with a buffer solution to remove the unbound antibody. The amount of the antibody bound onto the solid phase support is measured by a well known method. The detection conditions for each measurement may be appropriately determined by those skilled in the art using a common test method.

There are exemplary methods as follows for detectably labeling an anti-PCA-1 antibody. According to one method, the antibody is bound to a type of enzyme, for example, an enzyme usable for enzyme immunoassay (EIA) (Voiler, A., "The Enzyme Linked Immunosorbent Assay" (ELISA), 1978, Diagnostic Horizons, 2: 1-7, Microbiological Associates Quarterly Publication, Walkersville. MD; Voiler, A., J. Clin. Pathol., 31: 507-520, 1978; Butier, J. E., Meth. Enzymol., 73: 482-523, 1981). An enzyme bindable to the antibody is reacted with an appropriate substrate, preferably a chromogenic substrate by, for example, a method for generating a chemical molecule detectable with a fluorescent assay performed using visualization means on a spectrometer. Examples of the enzyme usable for detectably labeling an antibody include peroxidase and alkaline phosphatase, but are not limited to these. This detection may also be achieved by colorimetry using a chromogenic substrate to the enzyme.

The detection of an anti-PCA-1 antibody may also be performed using various other methods. For example, the expression of the PCA-1 protein can be detected using radioimmunoassay (RIA) by labeling the antibody or a fragment thereof with a radioactive substance (see, for example, Weintrsub, B., Principles of Radioimmunoassays, Seventh Training Course on Radioligand Assay Techniques, The Endocrine Society, March 1986). A radioactive isotope can be detected using means such as a gamma counter or a scintillation counter, or by autoradiography.

An antibody can also be labeled with a fluorescent compound. Examples of the fluorescent compound used most commonly include fluorescein isothiocyanate, rhodamine, phycoerythrin, and fluorescamine. Similarly, an anti-PCA-1 antibody can be labeled using a bioluminescent compound. The presence of a bioluminescent compound is measured by detecting the presence of fluorescence. Bioluminescent compounds important for this labeling purpose are luciferine, luciferase, and aequorin.

In a specific embodiment of the present invention, the expression level of the PCA-1 protein in a body fluid sample can be analyzed by two-dimensional electrophoresis. The two-dimensional electrophoresis is known to those skilled in the art. On a first stage, a body fluid sample such as a serum sample or the like is mounted on an electrophoresis gel for isoelectric focusing separation, which is provided for separating protein based on the charge. A great number of first stage gel specimens including gel strips for separation based on the immobilized gradient or gel tubes for separation based on the carrier amphoteric electrolyte are usable. After the primary separation, the protein is transferred to a second stage gel, equilibrated and then separated using SDS-PAGE, which is provided for separating the protein based on the molecular weight thereof For comparing the serum samples taken from different subjects, a plurality of gels are prepared from the serum samples.

After the separation, the protein is transferred from the second stage gel onto a membrane which is commonly used for the Western blotting method. The Western blotting method and the following visualization of the protein are well known to those skilled in the art (Sambrook et al., Molecular Cloning: A laboratory manual, 2nd Ed., Vol. 3, 1989, Cold Spring Harbor). A standard method is usable, or this standard method may be modified as known in the art for identifying a particular type of protein, for example, highly basic, highly acidic or lipid-soluble protein, etc. (see, for example, Ausubel et al., Current Protocols in Molecular Biology, Green Publishing Associates and Wiley Interscience, N.Y). An antibody bindable to the PCA-1 protein is used for an incubation step as in the Western blotting analysis method. A second antibody specific to a first antibody is used by the Western blotting analysis method to visualize the protein reactable with the first antibody.

### 2. Assay for detecting an anti-PCA-1 autoantibody

In another embodiment, the present invention provides a method for detecting and/or quantifying an anti-PCA-1 autoantibody in a body fluid sample from a subject as a prostate cancer marker. The method according to the present invention is usable for diagnosing prostate cancer. This method can also predict the progress of prostate cancer by monitoring the level of the anti-PCA-1 autoantibody.

An anti-PCA-1 autoantibody in a body fluid sample from a subject can be detected by any of many methods. A representative method is immunoassay. Examples of the immunoassay include Western blotting, radioimmunoassay, ELISA, sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, protein A immunoassay and the like.

Such an immunoassay can be performed in various methods. One exemplary method for performing such an immunoassay comprises anchoring the PCA-1 protein to a solid phase support, and detecting the anti-PCA-1 antibody specific to the PCA-1 protein. The PCA-1 protein usable for an assay according to the present invention can be prepared by a recombinant DNA technology well known in the art. For example, DNA encoding the PCA-1 protein may be introduced into an appropriate expression vector by a gene recombinant technology to express the PCA-1 protein on a large scale. Preferably, fused protein capable of facilitating the labeling, immobilization or detection of PCA-1 is subjected to gene manipulation (see, for example, the technology described in Sambrook et al., Molecular Cloning: A Laboratory Manual, 1989, Cold Spring Harbor Press, Cold Spring Harbor, N.Y.). According to another method, the PCA-1 protein may be purified from a naturally occurring source. For example, the PCA-1 protein may be purified from prostate cancer cells using a protein separation technology well known in the art. Examples of such a purification technology include molecular sieve chromatography and/or ion exchange chromatography, but are not limited to these. In actuality, a microtiter plate is advantageously used as a solid state support for the PCA-1 protein.

The assay for detecting PCA-1 and the assay for detecting an anti-PCA-1 autoantibody according to the present invention are each a novel method usable independently for diagnosis and/or recuperation evaluation of prostate cancer. The present invention is not limited to such a form of use, and these assays can, for example, further improve the accuracy of diagnosis when combined with other prostate cancer diagnostic methods. Such other prostate cancer diagnostic methods include palpation, PSA examination, pathological examination using a prostate cancer biopsy sample (pathological T stage), gleason cancer score, ultrasonic examination, MRI, CT, bone scintillation and the like.

### (Diagnostic agent)

In one embodiment, the present invention provides a prostate cancer diagnostic agent (or composition) for detecting and/or quantifying the PCA-1 protein or a fragment thereof in a body fluid sample from a subject as a prostate cancer marker, wherein the diagnostic agent comprises an anti-PCA-1 antibody.

In another embodiment, the present invention also provides a prostate cancer diagnostic agent for detecting and/or quantifying a PCA-1 gene, PCA-1 mRNA, or a fragment thereof in a body fluid sample from a subject as a prostate cancer marker, wherein the diagnostic agent comprises a polynucleotide formed of a nucleotide sequence hybridizable with the nucleotide sequence of the PCA-1 gene, PCA-1 mRNA, or a fragment thereof under stringent hybridization conditions.

In still another embodiment, the present invention provides a prostate cancer diagnostic agent for detecting and/or quantifying an anti-PCA-1 autoantibody in a body fluid sample from a subject as a prostate cancer marker, wherein the diagnostic agent comprises PCA-1 or a fragment thereof

A diagnostic agent according to the present invention comprises a component for detecting, for example, the PCA-1 protein, the PCA-1 gene or the anti-PCA-1 autoantibody in a body fluid sample (e.g., blood, urine) of a subject.

### (1) Diagnostic agent using an anti-PCA-1 antibody

When, for example, the PCA-1 protein or a fragment thereof in a body fluid sample from a subject is detected and/or quantified by ELISA, the component for detection and/or quantification is formed of, for example, an antibody directed to an epitope of the PCA-1 protein usable for detecting and/or quantifying the level of the PCA-1 in the body fluid sample such as blood or urine. The antibody may be labeled with radioactive, fluorescent, colorimetric, or enzymatic labeling so as to be detectable as it is.

### (2) Diagnostic agent using a nucleic acid (or nucleotide) probe or primer

When, for example, a PCA-1 gene, PCA-1 mRNA, or a fragment thereof in a body fluid sample from a subject is detected and/or quantified by hybridization with a probe, the component for detection and/or quantification is formed of a probe or primer designed, for example, based on the nucleotide sequence of the PCA-1 gene. Such a probe or primer can be obtained by designing an arbitrary number of appropriate (sense or antisense) probes or primers from the nucleotide sequence of the PCA-1 gene represented by SEQ ID NO: 1. Diagnosis using such a diagnostic agent is specifically carried out by performing, for example, a method comprising the steps of: (a) bringing a body fluid sample derived from a subject into contact with a polynucleotide (probe) formed of a nucleotide sequence hybridizable with the nucleotide sequence of the PCA-1 gene, PCA-1 mRNA, or a fragment thereof under stringent hybridization conditions and (b) detecting and/or quantifying the hybridization of the polynucleotide and the PCA-1 gene, PCA-1 mRNA, or a fragment thereof in the sample.

By a diagnostic method using the diagnostic agent according to the present invention, DNA (or a fragment thereof), mRNA (or a fragment thereof) or the like of a PCA-1 gene in a biological sample derived from a subject is detected and/or quantified using the above-mentioned probe. The polynucleotide used as a probe may have a nucleotide sequence of, for example, at least 12 nucleotides, at least 15 nucleotides, at least 18 nucleotides, at least 21 nucleotides, at least 24 nucleotides, at least 27 nucleotides, at least 30 nucleotides or longer.

The hybridization can be performed by a known method or a method conformed thereto, for example, a method described in Molecular Cloning, Third Edition, J. Sambrook et al., Cold Spring Harbor Lab. Press, 2001. When a commercially available library is used, a method described in the attached user's manual is usable. Herein, the "stringent conditions" may be any of low-stringent conditions, medium-stringent conditions or high-stringent conditions. The "low-stringent conditions" are, for example, 5 × SSC, 5 × Denhart's solution, 0.5% SDS, 50% formamide, 32°C. The "medium-stringent conditions" are, for example, 5 × SSC, 5 × Denhart's solution, 0.5% SDS, 50% formamide, 42°C: The "high-stringent conditions" are, for example, 5 × SSC, 5 × Denhart's solution, 0.5% SDS, 50% formamide, 50°C. Under these conditions, as the temperature is higher, DNA having a higher homology is expected to be obtained efficiently. There are a plurality of factors which are considered to influence the stringency of hybridization, for example, temperature, probe concentration, probe length, ion intensity, time, salt concentration and the like. Those skilled in the art would realize such stringency as any of the above-described conditions by appropriately selecting these factors.

As used herein, the "nucleotide sequence hybridizable with the nucleotide sequence of the PCA-1 gene, PCA-1 mRNA or a fragment thereof under stringent hybridization conditions" encompasses a nucleotide sequence complementary to the nucleotide sequence of the PCA-1 gene, PCA-1 mRNA or a fragment thereof (i.e., antisense DNA). Methods for hybridization of a probe and a nucleic acid are known to those skilled in the art and are described in, for example, WO89/06698, EP-A0200362, USP 2,915,082, EP-A0063879, EP-A0173251, and EP-A0128018.

When using the diagnostic agent and the diagnostic method according to the present invention described above, a target sequence can be detected or quantified using a probe or a primer specific to the PCA-1 gene, PCA-1 mRNA or a fragment thereof with a known technique. Examples of such a known technique include Southern hybridization method, Northern hybridization method, RT-PCR method, PCR-SSCP method (Genomics, Vol. 5, pp. 874-879 (1989), Proceedings of the National Academy of Science of the United States of America, Vol. 86, pp. 2766-2770 (1989)), FISH method, DNA chip method, array CGH method and the like. Quantitative detection can be carried out by quantitative RT-PCR.

### (3) Diagnostic agent using a PCA-1 antigen

When, for example, an autoantibody is detected and/or quantified by ELISA, the component for detection and/or quantification is formed of, for example, means for detecting a target antigen being bound to a solid phase support and assuming a form of at least one type of, preferably a plurality of types of, PCA-1 antigen or an epitope thereof, and an anti-PCA-1 autoantibody binding to the target antigen. Such detection means is, for example, an antibody directed to a constant region of the anti-PCA-1 autoantibody (e.g., rabbit anti-human IgG antibody), and is labeled so as to be detectable as it is (labeled with, for example, radioactive, fluorescent, colorimetric or enzymatic labeling) or detected by a labeled secondary antibody (e.g., goat anti-rabbit antibody). This diagnostic agent is used for detecting a prostate cancer marker by any of the above-described immunological techniques.

### (Kit)

In one embodiment, the present invention provides a kit for detecting and/or quantifying the PCA-1 protein or a fragment thereof in a body fluid sample from a subject as a prostate cancer marker, wherein the kit comprises an anti-PCA-1 antibody. The kit is used for detecting a prostate cancer marker by any of the above-described immunological techniques.

In another embodiment, the present invention provides a kit for detecting and/or quantifying a PCA-1 gene, PCA-1 mRNA or a fragment thereof in a body fluid sample from a subject as a prostate cancer marker, wherein the kit comprises a nucleotide sequence hybridizable with the nucleotide sequence of the PCA-1 gene, PCA-1 mRNA, or a fragment thereof under stringent hybridization conditions. The kit is used for detecting a prostate cancer marker by any of the above-mentioned hybridization methods.

In still another embodiment, the present invention provides a kit for detecting and/or quantifying an anti-PCA-1 autoantibody in a body fluid sample from a subject as a prostate cancer marker, wherein the kit comprises PCA-1 or a fragment thereof. The kit is used for detecting a prostate cancer marker by any of the above-mentioned immunological techniques.

The kit in the first embodiment comprises a component for detecting and/or quantifying a PCA-1 antigen in a body fluid sample from a subject. When, for example, the PCA-1 protein is detected and/or quantified by ELISA, such a component is formed of, for example, an antibody directed to an epitope of the PCA-1 protein usable for detecting and/or quantifying the level of the PCA-1 protein in the body fluid sample such as blood or urine. The antibody may be labeled with radioactive, fluorescent, colorimetric, or enzymatic labeling so as to be detectable as it is. Alternatively, the kit may comprise a labeled secondary antibody.

The kit in the second embodiment comprises a component for detecting and/or quantifying a PCA-1 gene or a fragment thereof in a body fluid sample from a subject, or a transcription product (mRNA) of the gene or a fragment thereof Such a component is formed of, for example, a polynucleotide consisting of a nucleotide sequence hybridizable with the nucleotide sequence of the PCA-1 gene, PCA-1 mRNA or a fragment thereof under stringent hybridization conditions. For example, the kit according to the present invention may comprise the above-mentioned polynucleotide immobilized on a DNA chip.

The kit in the third embodiment comprises a component necessary for detecting and/or quantifying an anti-PCA-1 autoantibody in a body fluid sample from a subject. When, for example, the autoantibody is detected and/or quantified by ELISA, such a component is formed of means for detecting a target antigen being bound to a solid phase support and assuming a form of at least one type of, preferably a plurality of types of, PCA-1 antigen or an epitope thereof, and an anti-PCA-1 autoantibody binding to the target antigen. Such detection means is, for example, an antibody (for example, rabbit anti-human IgG antibody) directed to a constant region of the anti-PCA-1 autoantibody, and is labeled (with, for example, radioactive, fluorescent, colorimetric or enzymatic labeling) so as to be detectable as it is or detected by a labeled secondary antibody (e.g., goat anti-rabbit antibody).

A kit according to the present invention may include a vessel, a label or the like in addition to a polynucleotide consisting of a nucleotide sequence hybridizable with the nucleotide sequence of the anti-PCA-1 antibody, PCA-1 gene, PCA-1 mRNA or a fragment thereof under stringent hybridization conditions, a PCA-1 antigen, an antibody against the anti-PCA-1 autoantibody, and the like. The label on, or accompanying, the vessel may indicate that the agent is used for detecting a prostate cancer marker. The kit may further comprise other items, for example, an instructions manual, a labeled secondary antibody or the like.

Hereinafter, the present invention will be described in more detail by way of examples. The present invention is not limited to these examples.

### EXAMPLES

### (Example 1)

### (Detection of PCA-1 in the blood using a mass spectrometer)

### 1. Pre-treatment of the sample

### (Removal of blood cell, albumin, IgG, etc.)

Serum derived from a prostate cancer patient was prepared by keeping still the blood, immediately after the blood was sampled, at room temperature for 40 minutes before removing blood cell components by centrifugation 12.5 µl of the sample (serum) was added to, and mildly mixed with, 50 µl of the Blue Sepharose 6 Fast Flow resin (Amersham Biosciences) which had been equilibrated in advance with 100 mM ammonium hydrogen carbonate. The mixture was kept still. 5 minutes later, the serum was transferred together with the Blue Sepharose 6 Fast Flow resin to a mini column (Wizard Minicolumns, Promega) set to a 1.5 ml microtube, and albumin was separated and removed by centrifugation.

Next, the fraction recovered to the 1.5 ml microtube was added to, and mildly mixed with, 50 µl of the Protein G Sepharose 4 Fast Flow resin (Amersham Biosciences) which had been equilibrated in advance with 100 mM ammonium hydrogen carbonate. The mixture was kept still. IgG was separated and removed by centrifugation in such manner as described above.

The final volume of the fraction obtained by these operations was about 65 µl.

### (Trypsin treatment)

25 µl was taken from the fraction obtained by the above-described treatment and mixed with 25 µl of 100 mM ammonium hydrogen carbonate. 0.5 µg of trypsin was added thereto, and the resultant substance was kept warm at 37°C overnight. In the case where the measurement was not performed immediately, the substance was kept at -80°C.

### 2. Detection of PCA-1 in the blood

In this example, a mass spectrometer coupled with an HPLC apparatus was used. The mass spectrometer ionizes components in the sample separated by HPLC and measures the mass of the ionized components. Specifically, HCTplus (Bruker Daltonics), which is an ion trap type liquid chromatograph/mass spectrometer (LC/MS) equipped with a nanoESI ion source as an ion source and PicoTip needle (inner diameter of the main body: 20 µm; inner diameter of the tip: 10 µm) (LC Packings) as a glass capillary, was used at a capillary voltage of 1.2 kV to 1.5 kV As a pre-column, a 0.3 × 10 mm column, equilibrated with 2% acetonitrile containing 0.1% formic acid, was used at a flow rate of 30 µL/min. As a main column, a 0.075 × 150 mm column was used at a flow rate of 200 nL/min. As an eluate of the sample, eluate A (2% acetonitrile containing 0.1 % formic acid) and eluate B (80% acetonitrile containing 0.1% formic acid) were used in accordance with the following gradient: 0/0-5/0-90/50-95/100-100/100-100.1/0-120/0 (min./B%).

A sample treated with trypsin as described above was set to a sample holder of the LC column of the above-described mass spectrometer, and the measurement was started.

### (Results)

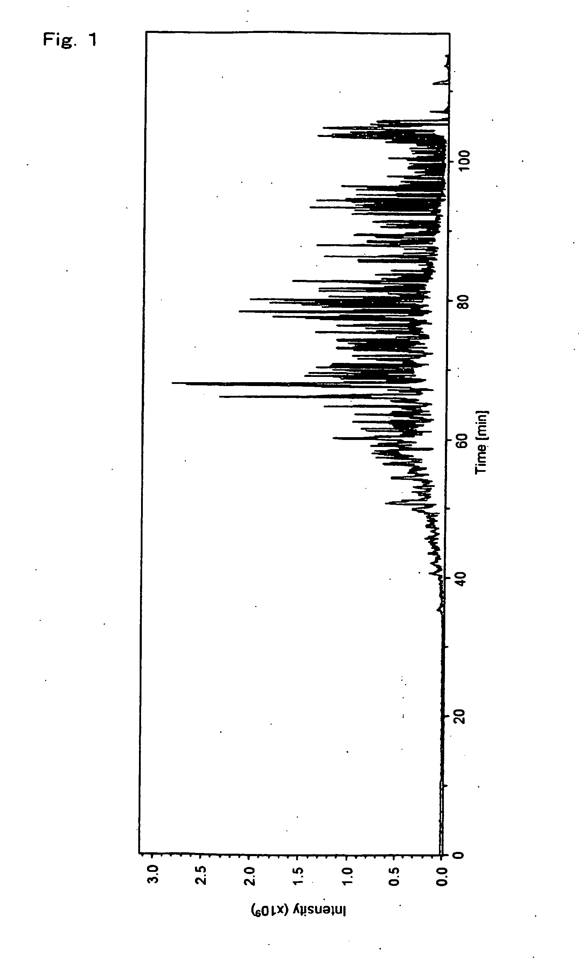

Fig. 1 shows an elution profile of a blood sample derived from a subject having prostate cancer, which was monitored by the LC-MS mass spectrometer. The vertical axis of the graph represents the ion intensity, and the horizontal axis represents the retention time. As shown here, the blood sample exhibits many peaks of the trypsin-degraded peptide fragment. Assuming that PCA-1 is present in the blood sample from the prostate cancer patient, at least one of the peaks is considered to be of a trypsin-degraded peptide fragment derived from PCA-1.

In order to check whether or not at least one of the peaks is of a trypsin-degraded peptide fragment derived from PCA-1, the present inventors attempted to calculate a mass of the trypsin-degraded peptide fragment unique to PCA-1 based on the amino acid sequence thereof and selectively monitor peptide fragments having such a specific mass.

Table 1 shows trypsin-degraded peptide fragments of PCA-1 obtained by the *in silico* calculation (peptides having a mass of 600 or greater), expected mass, expected divalent ion mass to charge ratio (m/z), and the position of each peptide fragment in the amino acid sequence of PCA-1.

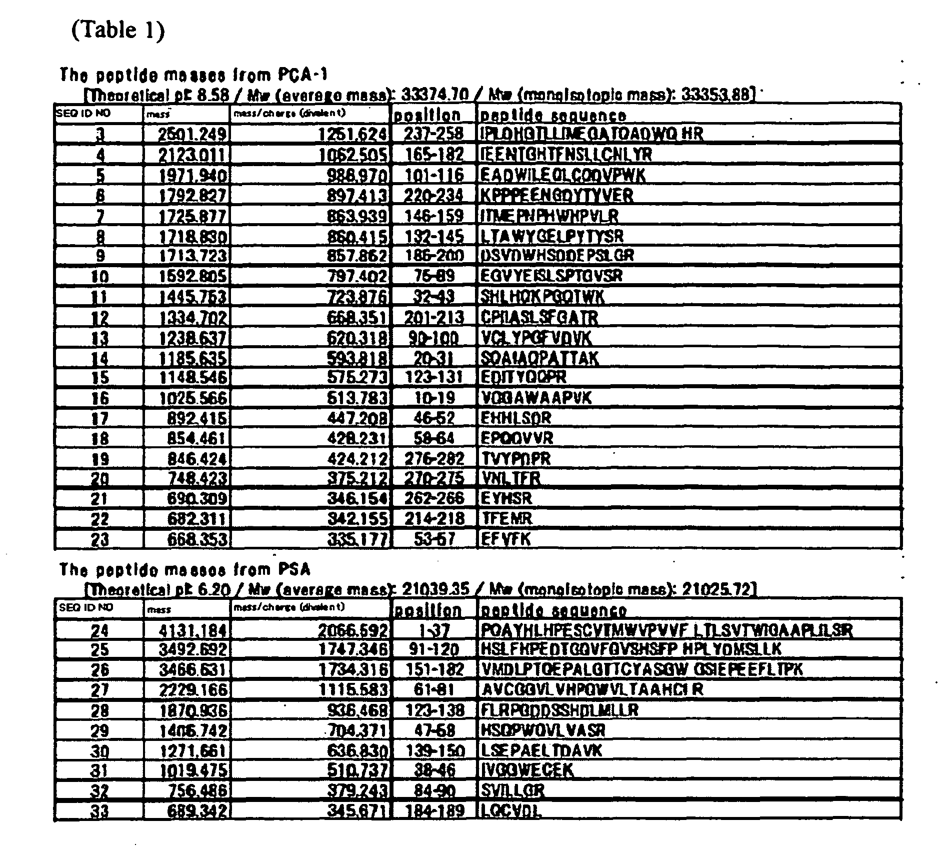

In addition to the mass of the peptide eluted from HPLC, the above-described mass spectrometer can measure the mass of various lengths of fragmented peptides (cleaved peptides) generated from cleavage of the peptide by providing excessive energy to the peptide when ionizing the peptide. By analyzing the difference in the mass of these cleaved peptides, the amino acid sequence forming the peptide can be determined.

It is considered that this principle can be utilized to detect a specific peptide having a known amino acid sequence from a mixture of peptides having many different mass values, based on the mass of the amino acid sequence and the mass of the cleaved peptides which can be generated from the specific peptide. Namely, it can be checked whether or not a molecule having a sequence unique to PCA-1 is present in the blood sample derived from a prostate cancer patient, based on the mass expected from the amino acid sequence of the trypsin-degraded peptide fragment of the PCA-1 and the mass of the cleaved peptides expected from the trypsin-degraded peptide fragment.

With such an idea, from the PCA-1 peptide fragments shown in Table 1, a peptide fragment of amino acid positions 75 to 89 of PCA-1: EGVYEISLSPTGVSR (SEQ ID NO: 10) and a peptide fragment of amino acid positions 10 to 19 of PCA-1: VQGAWAAPVK (SEQ ID NO: 16) were arbitrarily selected. Molecules having the mass of cleaved peptides expected from the mass which was drawn from the amino acid sequences of these molecules of the peptide fragments (1592.805 and 1025.566, respectively) were selectively monitored. Fig. 2 (parts A and B) shows the elution profiles thereof.

As shown in Fig. 2, part A, when the measurement conditions were set so as to selectively capture the mass to charge ratio for a divalent molecule expected from the peptide of the mass of SEQ ID NO: 10 (i.e., 797.4 m/z) and the mass to charge ratio for a monovalent molecule of a cleaved peptide which can be generated from the peptide of SEQ ID NO: 10 (i.e., 703 m/z), a single strong peak was observed around the retention time of 68 minutes (indicated by an arrow in the figure). This strongly suggests that a peptide having a sequence unique to PCA-1 is present in the sample. As shown in Fig. 2, part B, when the measurement conditions were set so as to selectively capture the mass to charge ratio for a divalent molecule expected from the mass of the peptide of SEQ ID NO: 16 (i.e., 513.8 m/z) and the mass to charge ratio for a monovalent molecule of a cleaved peptide which can be generated from the peptide of SEQ ID NO: 16 (i.e., 799 m/z), a single strong peak was observed around the retention time of 62 minutes (indicated by an arrow in the figure). This also strongly suggests that a peptide having a sequence unique to PCA-1 is present in the sample.

For comparison, using the sample from the same subject, the mass to charge ratio for a divalent molecule expected from the peptide unique to PSA: HSQPWQVLVASR (SEQ ID NO: 29 in Table 1) (i.e., 704.3 m/z) and the mass to charge ratio for a monovalent molecule of two cleaved peptides which can be generated from the peptide of SEQ ID NO: 29 (i.e., 695 m/z and 1055 m/z) were selectively monitored. As a result, for the ratio of 695 m/z, peaks were observed around the retention time of 52 minutes, 59 minutes and 64 minutes (Fig. 2, part C). For the ratio of 1055 m/z, a single peak was observed around the retention time of 64 minutes (Fig. 2, part D). These results suggest that the peak around 64 minutes (indicated by an arrow in the figure) is of the peptide unique to PSA having a mass to charge ratio of 704.3 m/z in the case of divalent (HSQPWQVLVASR), and also strongly suggest that this peptide is present in the blood sample from the subject.

### 3. Data analysis by MSMS

The above-described results indicate that molecules having a mass calculated based on the combination of specific amino acid residues are present in the sample, but the results are not sufficient enough to demonstrate that a peptide having a specific sequence is present in the sample.

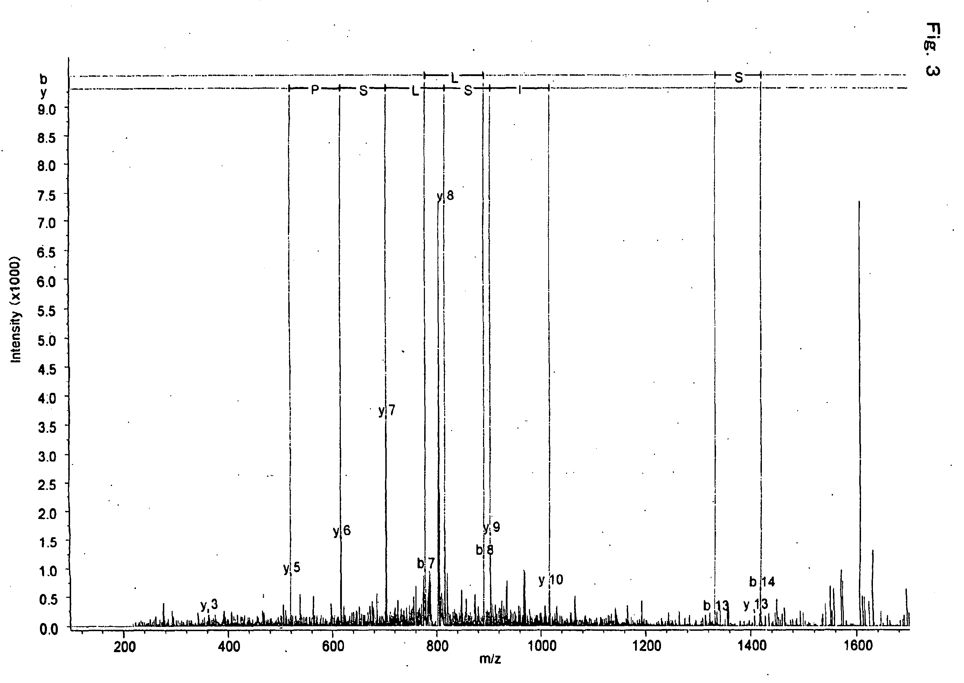

Therefore, MSMS data analysis was performed on the single peak observed in Fig. 2, part A (indicated by an arrow in the figure). The results are shown in Fig. 3. As shown here, strong signals are recognized at y5, y6, y7, y8, y9, y10 and the like. Based on the width between the signals, the mass was estimated. Amino acid residues corresponding to the mass were determined. As a result, a partial sequence of at least PSLSI from the C terminus was determined. This sequence matches a part of the peptide fragment of SEQ ID NO: 10. Based on this, it was confirmed that the peptide fragment of SEQ ID NO: 10 was present in the sample.

The names of the cleaved ions such as y5, y6 and the like are based on the system represented by the following chemical formula.

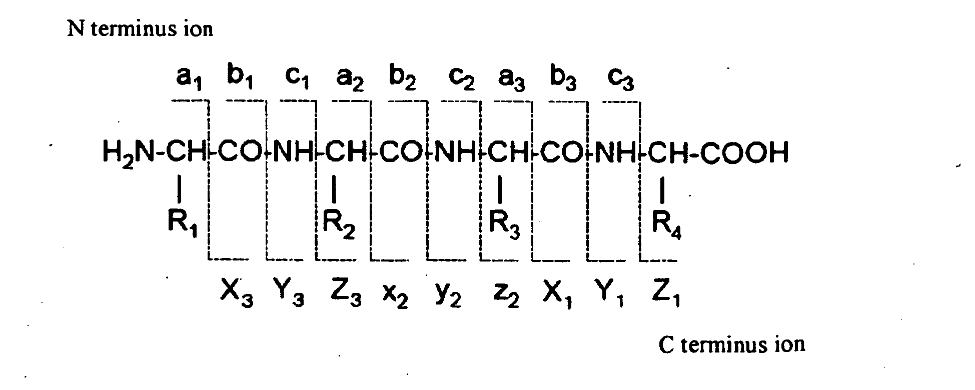

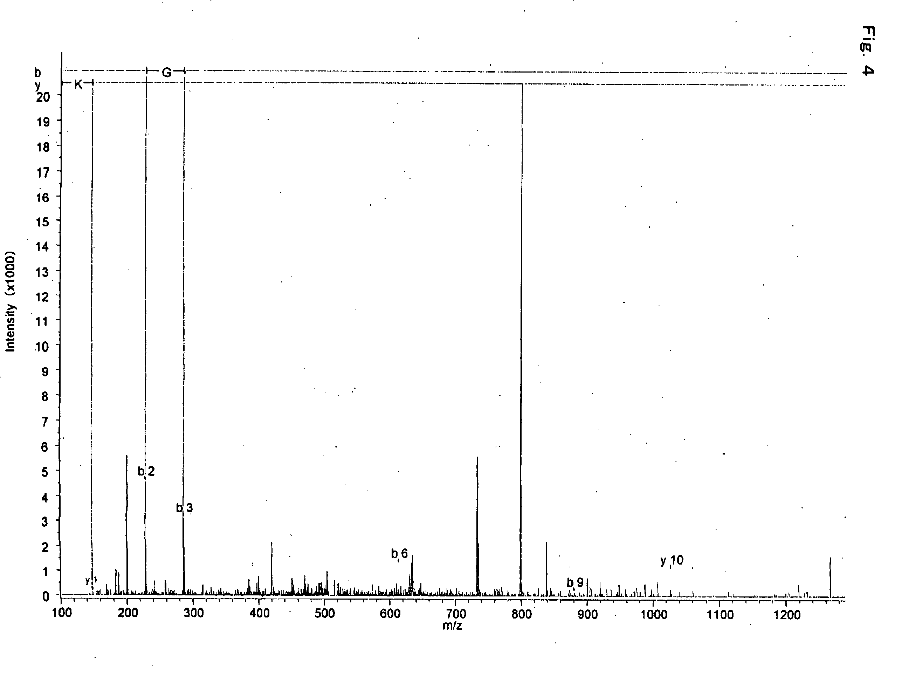

Fig. 4 shows the results of MSMS data analysis performed on the single peak observed in Fig. 2, part B (indicated by an arrow in the figure). As shown in Fig. 4, no appropriate sequence determination was possible in this case.

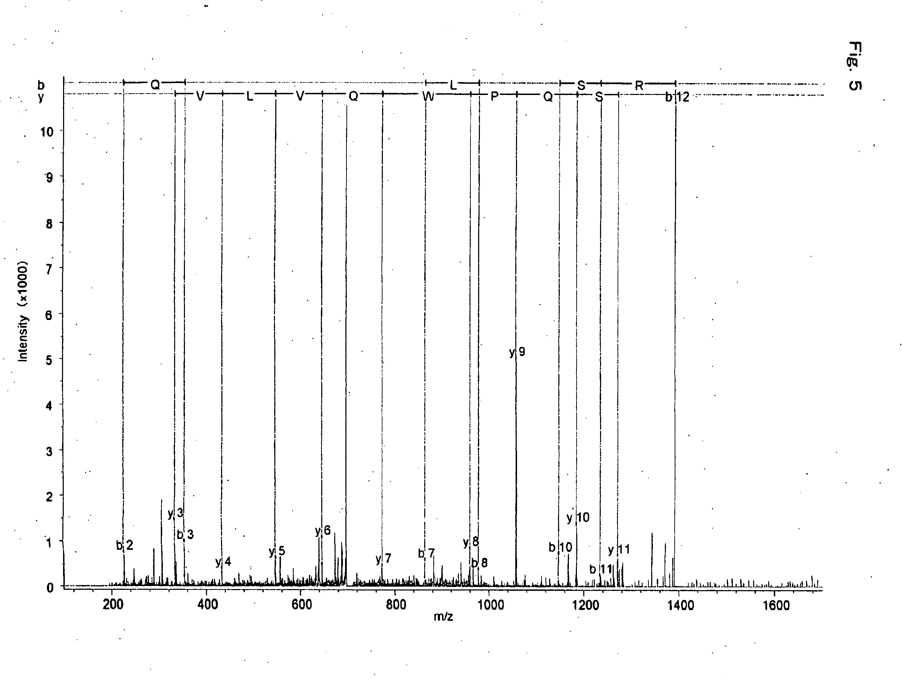

Fig. 5 shows the results of MSMS data analysis performed, for comparison, on the peak around the retention time of 64 minutes observed in Fig. 2, parts C and D. As shown in Fig. 5, strong signals were observed at y3, y4, y5, y6, y7, y8, y9, y10, y11 and the like. Based on the width between the signals, the mass was estimated. Amino acid residues corresponding to the mass were determined. As a result, a partial sequence of at least VLVQWPQS from the C terminus was determined. This sequence matches a part of the peptide fragment of SEQ ID NO: 29. Based on this, it was confirmed that the peptide fragment of SEQ ID NO: 29 was present in the sample.

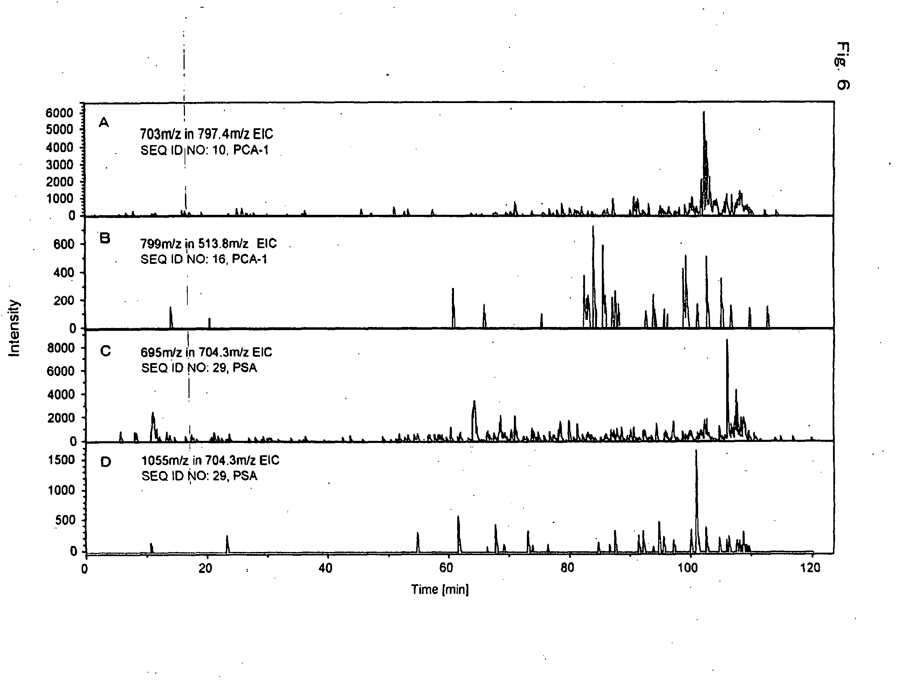

As a comparative example to the experiment shown in Fig. 2, Fig. 6 shows elution profiles for a blood sample derived from a healthy subject which was pre-treated and trypsin-treated. Fig. 6 shows the results obtained when molecules having a specific mass were monitored in the same measurement conditions as in the experiment shown in Fig. 2. As shown here, no highly strong peaks were observed. This shows that molecules having a mass corresponding to the expected mass of the trypsin-degraded peptide fragment of PCA-1 or the expected mass of the trypsin-degraded peptide fragment of PSA are present in too small an amount to be detected by the detection method developed by the present inventors using an LS-MS mass spectrometer, or such molecules are not present, in the blood sample from the healthy subject.

Thus, the method developed by the present inventors for detecting a specific sequence in a blood sample using an LC-MS mass spectrometer demonstrated that PCA-1 is present in the blood derived from a prostate cancer patient and is not, detectably present in the blood derived from a healthy subject.

This indicates that PCA-1 can be used as a prostate cancer marker in the blood, like PSA. These results strongly suggest that PCA-1 protein itself can be an autoantibody in a prostate cancer patient. Those skilled in the art would understand that an autoantigen against PCA-1 is also usable as a prostate cancer marker in the blood.

### (Example 2)

### (Detection of PCA-1 in the urine by Western blotting)

### (Procedure)

16 µl of urine samples (two samples from healthy subjects and two samples from prostate cancer patients) was subjected to electrophoresis using 12% NuPAGE Novex Bis-Tris Gel and NuPAGE Novex Bis-Tris electrophoresis systems (Invitrogen). After the electrophoresis, the protein was transcripted to a PVDF (polyvinylidene fluoride) membrane (BioRad) using a semi-drive blotting apparatus (BioRad). The PVDF membrane was blocked using 3% bovine serum albumin-phosphate buffered saline at 4°C for 24 hours. The resultant membrane was washed 3 times with TBS-T (20 mM Tris-HCl, pH8.0, 137 mM NaCl, 0.1% Tween 20), then incubated with an anti-PCA-1 polyclonal antibody (1 µg/ml) at room temperature for 1 hour, and washed 3 times with TBS-T. In order to detect antigen-antibody binding, the membrane was incubated at room temperature for 1 hour in 0.1 µg/mg HRP (horseradish peroxidase) crosslinked rabbit IgG (Santa Cruz Biotech) diluted with TBS-T. The resultant membrane was washed 3 times with TBS-T, and then chemical luminescence was caused using ECL Plus Western Blotting Detection System (Amersham Biosciences). The chemical luminescence was detected using Light-Capture (ATTO).

### (Results)

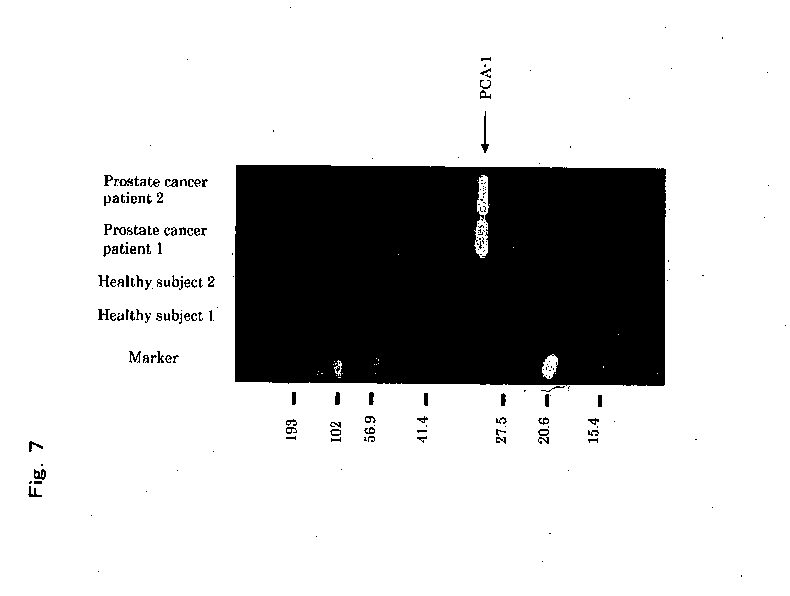

Fig. 7 shows the results. As shown here, in the urine samples from the prostate cancer patients, a conspicuous single band detectable with the anti-PCA-1 antibody was recognized. As a result of the analysis using a marker, the position of the band matched to the estimated molecular weight of the PCA-1 protein. In the sample from one healthy subject, a band was recognized, although weak, at the same position as the band detected in the urine samples from the prostate cancer patients, whereas intensity of the band was below the detection limit in the urine sample of the other healthy subject.

PCA-1 was detected in trace amounts or was not substantially detected in the urine samples from the healthy subjects, but was conspicuously detected in the urine samples derived from the prostate cancer patients. These results indicate that PCA-1 is usable as a prostate cancer marker in the urine.

### INDUSTRIAL APPLICABILITY

The present invention provides a prostate cancer marker useful for diagnosing prostate cancer accurately, rapidly and simply while alleviating the burden on both of the patients and physicians.

## Claims

1. A method for diagnosing prostate cancer comprising detecting and/or quantifying PCA-1 in a body fluid sample derived from a subject as a prostate cancer marker.

2. The method according to claim 1, wherein the body fluid sample is
a) whole blood, serum or plasma, or
b) urine.

3. The method according to claim 1 or 2, wherein the PCA-1 is detected and/or quantified using a mass spectrometer.

4. The method according to claim 1 or 2, wherein the PCA-1 is detected and/or quantified using an anti-PCA-1 antibody.

5. The method according to claim 4, comprising the steps of:
bringing the body fluid sample into contact with the anti-PCA-1 antibody; and
detecting and/or quantifying the binding between the PCA-1 and the anti-PCA-1 antibody in the body fluid sample.

6. The method according to claim 5, wherein the step of detecting and/or quantifying comprises detecting and/or quantifying the binding between the PCA-1 and the anti-PCA-1 antibody using a labeled anti-PCA-1 antibody.

7. The method according to any one of claims 4 through 6, which is performed in accordance with an immunoassay selected from the group consisting of Western blotting, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, and protein A immunoassay.

8. Use of An anti-PCA-1 antibody as a prostate cancer diagnostic agent on a body fluid sample.

9. Use of A polynucleotide formed of a nucleotide sequence hybridizable with the nucleotide sequence of the PCA-1 gene, or PCA-1 mRNA under stringent hybridization conditions as a prostate cancer diagnostic agent on a body fluid sample.

10. The use according to claim 8 or claim 9 wherein the body fluid sample is
a) whole blood, serum or plasma, or
b) urine.

11. Use of a kit for the diagnosis of prostate cancer, wherein the use is on a body fluid sample, the kit comprising an anti-PCA-1 antibody.

12. Use according to claim 11, the kit further comprising a labeled anti-PCA-1 antibody.

13. Use according to claim 11 or 12, wherein detection and/or quantification of PCA-1 is performed in accordance with an immunoassay selected from the group consisting of Western blotting, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, and protein A immunoassay.

14. Use of a kit for the diagnosis of prostate cancer, wherein the use is on a body fluid sample, the kit comprising a polynucleotide formed of a nucleotide sequence hybridizable with the nucleotide sequence of the PCA-1 gene, or PCA-1 mRNA under stringent hybridization conditions.

15. Use of a kit according to any one of claims 11 through 14, wherein the body fluid sample is
a) whole blood, serum or plasma, or
b) urine.

16. A method for diagnosing prostate cancer comprising detecting and/or quantifying an anti-PCA-1 autoantibody in a body fluid sample derived from a subject as a prostate cancer marker.

17. The method according to claim 16, wherein the body fluid sample is
a) whole blood, serum or plasma, or
b) urine.

18. The method according to any one of claims 16 or 17, wherein the anti-PCA-1 autoantibody is detected and/or quantified using a PCA-1 antigen.

19. The method according to claim 18, comprising the steps of:
bringing the body fluid sample into contact with the PCA-1 antigen; and
detecting and/or quantifying the binding between the anti-PCA-1 autoantibody and the PCA-1 antigen in the body fluid sample.

20. The method according to claim 19, wherein the step of detecting and/or quantifying comprises detecting and/or quantifying the binding between the PCA-1 and the anti-PCA-1 autoantibody using a labeled antibody against the anti-PCA-1 autoantibody.

21. The method according to any one of claims 16 through 20, which is performed in accordance with an immunoassay selected from the group consisting of Western blotting, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, and protein A immunoassay.

22. Use of a kit for the diagnosis of prostate cancer, wherein the use is on a body fluid sample the kit comprising a PCA-1 antigen.

23. Use according to claim 22, the kit further comprising a labeled antibody against the anti-PCA-1 autoantibody so as to use the labeled antibody for detecting and/or quantifying the binding between the PCA-1 antigen and the anti-PCA-1 autoantibody.

24. Use according to claim 22 or 23, wherein detection and/or quantification of an anti-PCA-1 antibody is performed in accordance with an immunoassay selected from the group consisting of Western blotting, radioimmunoassay (RIA), enzyme-linked immunosorbent assay (ELISA), sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, and protein A immunoassay.

## Patentansprüche

1. Verfahren zur Diagnose von Prostatakrebs, welches den Schritt der Erfassung und/ oder quantitativen Bestimmung des Prostatakrebs-Antigen-1 bzw. PCA-1 in einer Körperflüssigkeitsprobe aufweist, welche von einer Testperson als Prostatakrebs-Marker stammt.

2. Verfahren nach Anspruch 1, **dadurch gekennzeichnet, dass** es sich bei der Körperflüssigkeitsprobe um Folgendes handelt:
a) Vollblut, Blutserum oder Plasma, oder
b) Urin.

3. Verfahren nach Anspruch 1 oder 2, **dadurch gekennzeichnet, dass** das PCA-1 durch Verwendung eines Massenspektrometers erfasst und/oder quantitativ bestimmt wird.

4. Verfahren nach Anspruch 1 oder 2, **dadurch gekennzeichnet, dass** das PCA-1 durch Verwendung eines Anti-PCA-1-Antikörpers erfasst und/oder quantitativ bestimmt wird.

5. Verfahren nach Anspruch 4, welches die folgenden Schritte aufweist:
Inkontaktbringen der Körperflüssigkeitsprobe mit dem Anti-PCA-1-Antikörper; und
Erfassen und/oder quantitatives Bestimmen der Bindung zwischen dem PCA-1 und dem Anti-PCA-1-Antikörper in der Körperflüssigkeitsprobe.

6. Verfahren nach Anspruch 5, **dadurch gekennzeichnet, dass** der Schritt der Erfassung und/oder quantitativen Bestimmung den Schritt der Erfassung und/oder quantitativen Bestimmung der Bindung zwischen dem PCA-1 und dem Anti-PCA-1-Antikörper durch Verwendung eines gekennzeichneten bzw. markierten Anti-PCA-1-Antikörpers aufweist.

7. Verfahren nach einem der Ansprüche 4 bis 6, welches gemäß einem Immuntest oder Immunoassay durchgeführt wird, welcher aus der Gruppe ausgewählt wird, welche besteht aus: Western Blotting, Radioimmunoassay (RIA), enzymgekoppelter Immunadsorptionstest (ELISA), Sandwich-Immunoassay, Fluoreszenz-Immunoassay (FIA), Time-resolved Fluoreszenz-Immunoassay (TRFIA), Enzym-Immunoassay (EIA), Lumineszenz-Immunoassay (LIA), elektrochemischer Lumineszenz-Immunoassay (ECLIA), Latex-Aggregation, Immunopräzipitations-Assay, Präzipitationsreaktion, Geldiffusions-Präzipitationsreaktion, Immunodiffusions-Assay, Agglutinations-Assay, Komplementfixations-Assay bzw. Komplementbindungstest, immunradiometrischer Assay, Fluoroimmunoassay, und Protein A-Immunoassay.

8. Verwendung eines Anti-PCA-1-Antikörpers als Prostatakrebs-Diagnosestoff bei einer Körperflüssigkeitsprobe.

9. Verwendung eines Polynukleotids, welches aus einer Nukleotidsequenz gebildet ist, welche mit der Nukleotidsequenz des PCA-1-Gens, oder der PCA-1 mRNA unter strengen Kreuzungs- bzw. Hybridisierungsvoraussetzungen als ein Prostatakrebs-Diagnosestoff bei einer Körperflüssigkeitsprobe gekreuzt bzw. hybridisiert werden kann.

10. Verwendung gemäß Anspruch 8 oder Anspruch 9, **dadurch gekennzeichnet, dass** es sich bei der Körperflüssigkeitsprobe um Folgendes handelt:
a) Vollblut, Blutserum oder Plasma, oder
b) Urin.

11. Verwendung einer Ausrüstung zur Diagnose von Prostatakrebs, **dadurch gekennzeichnet, dass** die Verwendung an einer Körperflüssigkeitsprobe erfolgt, wobei die Ausrüstung einen Anti-PCA-1-Antikörper aufweist.

12. Verwendung nach Anspruch 11, wobei die Ausrüstung des Weiteren einen gekennzeichneten bzw. markierten Anti-PCA-1-Antikörper aufweist.

13. Verwendung nach Anspruch 11 oder 12, **dadurch gekennzeichnet, dass** eine Erfassung und/oder quantitative Bestimmung des PCA-1 gemäß einem Immuntest bzw. Immunoassay durchgeführt wird, welcher aus der Gruppe ausgewählt wird, welche besteht aus: Western Blotting, Radioimmunoassay (RIA), enzymgekoppelter Immunadsorptionstest (ELISA), Sandwich-Immunoassay, Fluoreszenz-Immunoassay (FIA), Time-resolved Fluoreszenz-Immunoassay (TRFIA), Enzym-Immunoassay (EIA), Lumineszenz-Immunoassay (LIA), elektrochemischer Lumineszenz-Immunoassay (ECLIA), Latex-Aggregation, Immunopräzipitations-Assay, Präzipitationsreaktion, Geldiffusions-Präzipitationsreaktion, Immunodiffusions-Assay, Agglutinations-Assay, Komplementfixations-Assay bzw. Komplementbindungstest, immunradiometrischer Assay, Fluoroimmunoassay, und Protein A-Immunoassay.

14. Verwendung einer Ausrüstung für die Diagnose von Prostatakrebs, **dadurch gekennzeichnet, dass** die Verwendung an einer Körperflüssigkeitsprobe erfolgt, wobei die Ausrüstung ein Polynukleotid aufweist, welches aus einer Nukleotidsequenz gebildet ist, welche mit der Nukleotidsequenz des PCA-1-Gens, oder der PCA-1mRNA unter strengen Kreuzungs- bzw. Hybridisierungsvoraussetzungen gekreuzt bzw. hybridisiert werden kann.

15. Verwendung einer Ausrüstung gemäß einem der Ansprüche 11 bis 14, **dadurch gekennzeichnet, dass** es sich bei der Körperflüssigkeitsprobe um Folgendes handelt:
a) Vollblut, Blutserum oder Plasma, oder
b) Urin.

16. Verfahren zur Diagnose von Prostatakrebs, welches den Schritt der Erfassung und/ oder quantitativen Bestimmung eines Anti-PCA-1-Autoantikörpers in einer Körperflüssigkeitsprobe aufweist, welche von einer Testperson als ein Prostatakrebs-Marker stammt.

17. Verfahren nach Anspruch 16, **dadurch gekennzeichnet, dass** es sich bei der Körperflüssigkeitsprobe um Folgendes handelt:
a) Vollblut, Blutserum oder Plasma, oder
b) Urin.

18. Verfahren nach einem der Ansprüche 16 oder 17, **dadurch gekennzeichnet, dass** der Anti-PCA-1-Autoantikörper durch Verwendung eines PCA-1-Antigens erfasst und/ oder quantitativ bestimmt wird.

19. Verfahren nach Anspruch 18, welches die folgenden Schritte aufweist:
Inkontaktbringen der Körperflüssigkeitsprobe mit dem PCA-1-Antigen; und
Erfassen und/oder quantitatives Bestimmen der Bindung zwischen dem Anti-PCA-1-Autoantikörper und dem PCA-1-Antigen in der Körperflüssigkeitsprobe.

20. Verfahren nach Anspruch 19, **dadurch gekennzeichnet, dass** der Schritt der Erfassung und/oder quantitativen Bestimmung den Schritt der Erfassung und/oder quantitativen Bestimmung der Bindung zwischen dem PCA-1 und dem Anti-PCA-1-Autoantikörper unter Verwendung eines gekennzeichneten bzw. markierten Antikörpers gegen den Anti-PCA-1-Autoantikörper aufweist.

21. Verfahren nach einem der Ansprüche 16 bis 20, welches gemäß einem Immuntest bzw. Immunoassay durchgeführt wird, welcher aus der Gruppe ausgewählt wird, welche besteht aus: Western Blotting, Radioimmunoassay (RIA), enzymgekoppelter Immunadsorptionstest (ELISA), Sandwich-Immunoassay, Fluoreszenz-Immunoassay (FIA), Time-resolved Fluoreszenz-Immunoassay (TRFIA), Enzym-Immunoassay (EIA), Lumineszenz-Immunoassay (LIA), elektrochemischer Lumineszenz-Immunoassay (ECLIA), Latex-Aggregation, Immunopräzipitations-Assay, Präzipitationsreaktion, Geldiffusions-Präzipitationsreaktion, Immunodiffusions-Assay, Agglutinations-Assay, Komplementfixations-Assay bzw. Komplementbindungstest, immunradiometrischer Assay, Fluoroimmunoassay, und Protein A-Immunoassay.

22. Verwendung einer Ausrüstung zur Diagnose von Prostatakrebs, **dadurch gekennzeichnet, dass** die Verwendung an einer Körperflüssigkeitsprobe erfolgt, wobei die Ausrüstung ein PCA-1-Antigen aufweist.

23. Verwendung gemäß Anspruch 22, wobei die Ausrüstung des Weiteren einen gekennzeichneten bzw. markierten Antikörper gegen den Anti-PCA-1-Autoantikörper aufweist, so dass der gekennzeichnete bzw. markierte Antikörper zur Erfassung und/ oder quantitativen Bestimmung der Bindung zwischen dem PCA-1-Antigen und dem Anti-PCA-1-Autoanitkörper verwendet wird.

24. Verwendung nach Anspruch 22 oder 23, **dadurch gekennzeichnet, dass** eine Erfassung und/oder quantitative Bestimmung eines Anti-PCA-1-Antikörpers gemäß einem Immuntest bzw. Immunoassay durchgeführt wird, welcher aus der Gruppe ausgewählt wird, welche besteht aus: Western Blotting, Radioimmunoassay (RIA), enzymgekoppelter Immunadsorptionstest (ELISA), Sandwich-Immunoassay, Fluoreszenz-Immunoassay (FIA), Time-resolved Fluoreszenz-Immunoassay (TRFIA), Enzym-Immunoassay (EIA), Lumineszenz-Immunoassay (LIA), elektrochemischer Lumineszenz-Immunoassay (ECLIA), Latex-Aggregation, Immunopräzipitations-Assay, Präzipitationsreaktion, Geldiffusions-Präzipitationsreaktion, Immunodiffusions-Assay, Agglutinations-Assay, Komplementfixations-Assay bzw. Komplementbindungstest, immunradiometrischer Assay, Fluoroimmunoassay, und Protein A-Immunoassay.

## Revendications

1. Procédé pour diagnostiquer le cancer de la prostate comprenant le fait de détecter et/ou quantifier la PCA-1 dans un échantillon de fluide corporel dérivé d'un sujet comme marqueur du cancer de la prostate.

2. Procédé selon la revendication 1, dans lequel l'échantillon de fluide corporel est
a) du sang total, du sérum ou un plasma, ou
b) de l'urine.

3. Procédé selon la revendication 1 ou 2, dans lequel la PCA-1 est détectée et/ou quantifiée en utilisant un spectromètre de masse.

4. Procédé selon la revendication 1 ou 2, dans lequel la PCA-1 est détectée et/ou quantifiée en utilisant un anticorps anti-PCA-1.

5. Procédé selon la revendication 4, comprenant les étapes qui consistent à :
amener l'échantillon de fluide corporel en contact avec l'anticorps anti-PCA-1 ; et
détecter et/ou quantifier la liaison entre la PCA-1 et l'anticorps anti-PCA-1 dans l'échantillon de fluide corporel.

6. Procédé selon la revendication 5, dans lequel l'étape de détection et/ou de quantification comprend le fait de détecter et/ou de quantifier la liaison entre la PCA-1 et l'anticorps anti-PCA-1 en utilisant un anticorps anti-PCA-1 marqué.

7. Procédé selon l'une quelconque des revendications 4 à 6, qui est exécuté en accord avec un immunoessai sélectionné du groupe constitué d'un buvardage de Western, un dosage radio-immunologique (RIA), un dosage d'immunosorption liée à l'enzyme (ELISA), un dosage immunologique en sandwich, un dosage immunologique par fluorescence (FIA), un dosage immunologique par fluorescence à résolution temporelle (TRFIA), un dosage enzymo-immunologique (EIA), un dosage immunologique par luminescence (LIA), un dosage immunologique par luminescence électrochimique (ECLIA), une agrégation des latex, un dosage immunologique par précipitation, une réaction à la précipitine, une réaction à la précipitine par diffusion de gel, un dosage immunologique par diffusion, un dosage d'agglutinat, un dosage par fixation du complément, un dosage radio immunométrique, un dosage fluoro-immunologique, et d'un dosage immunologique de la protéine A.

8. Utilisation d'un anticorps anti-PCA-1 comme agent de diagnostic du cancer de la prostate sur un échantillon de fluide corporel.

9. Utilisation d'un polynucléotide formé d'une séquence nucléotidique pouvant être hybridée avec la séquence nucléotidique du gène PCA-1, ou de l'ARNm PCA-1 dans des conditions d'hybridation stringentes comme agent de diagnostic du cancer de la prostate sur un échantillon de fluide corporel.

10. Utilisation selon la revendication 8 ou 9, dans laquelle l'échantillon de fluide corporel est
a) du sang total, du sérum ou un plasma, ou
b) de l'urine.

11. Utilisation d'un kit pour diagnostiquer le cancer de la prostate, dans laquelle l'utilisation est effectuée sur un échantillon de fluide corporel, le kit comprenant un anticorps anti-PCA-1.

12. Utilisation selon la revendication 11, le kit comprenant en plus un anticorps anti-PCA-1 marqué.

13. Utilisation selon la revendication 11 ou 12, dans laquelle la détection et/ou la quantification de la PCA-1 est effectuée en accord avec un dosage immunologique sélectionné du groupe constitué d'un buvardage de Western, un dosage radio-immunologique (RIA), un dosage d'immunosorption liée à l'enzyme (ELISA), un dosage immunologique en sandwich, un dosage immunologique par fluorescence (FIA), un dosage immunologique par fluorescence à résolution temporelle (TRFIA), un dosage enzymo-immunologique (EIA), un dosage immunologique par luminescence (LIA), un dosage immunologique par luminescence électrochimique (ECLIA), une agrégation des latex, un dosage immunologique par précipitation, une réaction à la précipitine, une réaction à la précipitine par diffusion de gel, un dosage immunologique par diffusion, un dosage d'agglutinat, un dosage par fixation du complément, un dosage radio immunométrique, un dosage fluoro-immunologique, et d'un dosage immunologique de la protéine A.

14. Utilisation d'un kit pour diagnostiquer le cancer de la prostate, dans laquelle l'utilisation est effectuée sur un échantillon de fluide corporel, le kit comprenant un polynucléotide formé d'une séquence nucléotidique pouvant être hybridée avec la séquence nucléotidique du gène PCA-1, ou de l'ARNm PCA-1 dans des conditions d'hybridation stringentes.

15. Utilisation d'un kit selon l'une quelconque des revendications 11 à 14, dans laquelle l'échantillon de fluide corporel est
a) du sang total, du sérum ou un plasma, ou
b) de l'urine.

16. Procédé pour diagnostiquer le cancer de la prostate comprenant le fait de détecter et/ou quantifier un anticorps anti-PCA-1 dans un échantillon de fluide corporel dérivé d'un sujet comme marqueur du cancer de la prostate.

17. Procédé selon la revendication 16, dans lequel l'échantillon de fluide corporel est
a) du sang total, du sérum ou un plasma, ou
b) de l'urine.

18. Procédé selon l'une quelconque des revendications 16 ou 17, dans lequel l'anticorps anti-PCA-1 est détecté et/ou quantifié en utilisant un antigène PCA-1.

19. Procédé selon la revendication 18, comprenant les étapes qui consistent à :
amener l'échantillon de fluide corporel en contact avec l'antigène PCA-1 ; et
détecter et/ou quantifier la liaison entre l'auto-anticorps anti-PCA-1 et l'antigène PCA-1 dans l'échantillon de fluide corporel.

20. Procédé selon la revendication 19, dans lequel l'étape de détection et/ou de quantification comprend le fait de détecter et/ou quantifier la liaison entre la PCA-1 et l'auto-anticorps anti-PCA-1 en utilisant un anticorps marqué contre l'auto-anticorps anti-PCA-1.

21. Procédé selon l'une quelconque des revendications 16 à 20, qui est exécuté en accord avec un dosage immunologique sélectionné du groupe constitué d'un buvardage de Western, un dosage radio-immunologique (RIA), un dosage d'immunosorption liée à l'enzyme (ELISA), un dosage immunologique en sandwich, un dosage immunologique par fluorescence (FIA), un dosage immunologique par fluorescence à résolution temporelle (TRFIA), un dosage enzymo-immunologique (EIA), un dosage immunologique par luminescence (LIA), un dosage immunologique par luminescence électrochimique (ECLIA), une agrégation des latex, un dosage immunologique par précipitation, une réaction à la précipitine, une réaction à la précipitine par diffusion de gel, un dosage immunologique par diffusion, un dosage d'agglutinat, un dosage par fixation du complément, un dosage radio immunométrique, un dosage fluoro-immunologique, et d'un dosage immunologique de la protéine A.

22. Utilisation d'un kit pour diagnostiquer le cancer de la prostate, dans laquelle l'utilisation est effectuée sur un échantillon de fluide corporel, le kit comprenant un antigène PCA-1.

23. Utilisation selon la revendication 22, le kit comprenant un anticorps marqué contre l'auto-anticorps anti-PCA-1 de façon à utiliser l'anticorps marqué pour détecter et/ou quantifier la liaison entre l'antigène PCA-1 et l'auto-anticorps anti-PCA1.

24. Utilisation selon la revendication 22 ou 23, dans laquelle la détection et/ou la quantification d'un anticorps anti-PCA-1 est effectuée en accord avec un dosage immunologique sélectionné du groupe constitué d'un buvardage de Western, un dosage radio-immunologique (RIA), un dosage d'immunosorption liée à l'enzyme (ELISA), un dosage immunologique en sandwich, un dosage immunologique par fluorescence (FIA), un dosage immunologique par fluorescence à résolution temporelle (TRFIA), un dosage enzymo-immunologique (EIA), un dosage immunologique par luminescence (LIA), un dosage immunologique par luminescence électrochimique (ECLIA), une agrégation des latex, un dosage immunologique par précipitation, une réaction à la précipitine, une réaction à la précipitine par diffusion de gel, un dosage immunologique par diffusion, un dosage d'agglutinat, un dosage par fixation du complément, un dosage radio immunométrique, un dosage fluoro-immunologique, et d'un dosage immunologique de la protéine A.


### SEQUENCE LISTING

<110> Link Genomics, Inc.
   PCA InterMed
   Osaka University
<120> Method for diagnosis of prostate cancer
<130> P94218EP00
<140> EP 06715708.1 <141> 2006-03-14
<150> PCT/JP2006/305480 <151> 2006-03-14
<150> JP 2005-071387 <151> 2005-03-14
<150> JP 2005-241414 <151> 2005-08-23
<160> 34
<170> PatentIn version 3.3
<210> 1
   <211> 1520
   <212> DNA
   <213> Homo sapiens
<220>
   <221> CDS
   <222> (407)..(1267)
<400> 1
   aggtcacaga ctgcggagtg ggtcaggggc tgcgagggct gccccaagtc ctaccgggtt      60
   tgcacgggcg cgcccggctc cgcccgcaag tgcgccttcc tgacttactg ctgggtgcgc     120
   ggggctgggg gtgcgagtac cacccctgaa gtctcttcct gggcgacctc cggggcctca     180
   ttctaggcct ccttaaagag aaggatctaa attaggaaaa ggaagtgccc ttatccacga     240
   ccaagctctt ccacctgcgg agctcgctta gtctgcacct caaccgtgcg gaaagtgact     300
   gccctgttta ctgaggaaaa actggggctc agaaagatac catggagtag tttgaaacag     360
   gaacaaaatc ttctgaaagc tcggagcaga agcctttttg gtcaac atg gag gaa        415
                                                      Met Glu Glu
                                                      1
   aaa aga cgg cga gcc cga gtt cag gga gcc tgg gct gcc cct gtt aaa       463
   Lys Arg Arg Arg Ala Arg Val Gln Gly Ala Trp Ala Ala Pro Val Lys
       5                   10                  15
   agc cag gcc att gct cag cca gct acc act gct aag agc cat ctc cac       511
   Ser Gln Ala Ile Ala Gln Pro Ala Thr Thr Ala Lys Ser His Leu His
   20                  25                  30                  35
   cag aag cct ggc cag acc tgg aag aac aaa gag cat cat ctc tct gac      559
   Gln Lys Pro Gly Gln Thr Trp Lys Asn Lys Glu His His Leu Ser Asp
                   40                  45                  50
   aga gag ttt gtg ttc aaa gaa cct cag cag gta gta cgt aga gct cct      607
   Arg Glu Phe Val Phe Lys Glu Pro Gln Gln Val Val Arg Arg Ala Pro
               55                  60                  65
   gag cca cga gtg att gac aga gag ggt gtg tat gaa atc agc ctg tca      655
   Glu Pro Arg Val Ile Asp Arg Glu Gly Val Tyr Glu Ile Ser Leu Ser
           70                  75                  80
   ccc aca ggt gta tct agg gtc tgt ttg tat cct ggc ttt gtt gac gtg      703
   Pro Thr Gly Val Ser Arg Val Cys Leu Tyr Pro Gly Phe Val Asp Val
       85                  90                  95
   aaa gaa gct gac tgg ata ttg gaa cag ctt tgt caa gat gtt ccc tgg      751
   Lys Glu Ala Asp Trp Ile Leu Glu Gln Leu Cys Gln Asp Val Pro Trp
   100                 105                 110                 115
   aaa cag agg acc ggc atc aga gag gat ata act tat cag caa cca aga      799
   Lys Gln Arg Thr Gly Ile Arg Glu Asp Ile Thr Tyr Gln Gln Pro Arg
                   120                 125                 130
   ctt aca gca tgg tat gga gaa ctt cct tac act tat tca aga atc act      847
   Leu Thr Ala Trp Tyr Gly Glu Leu Pro Tyr Thr Tyr Ser Arg Ile Thr
               135                 140                 145
   atg gaa cca aat cct cac tgg cac cct gtg ctg cgc aca cta aag aac      895
   Met Glu Pro Asn Pro His Trp His Pro Val Leu Arg Thr Leu Lys Asn
           150                 155                 160
   cgc att gaa gag aac act ggc cac acc ttc aac tcc tta ctc tgc aat      943
   Arg Ile Glu Glu Asn Thr Gly His Thr Phe Asn Ser Leu Leu Cys Asn
       165                 170                 175
   ctt tat cgc aat gag aag gac agc gtg gac tgg cac agt gat gat gaa      991
   Leu Tyr Arg Asn Glu Lys Asp Ser Val Asp Trp His Ser Asp Asp Glu
   180                 185                 190                 195
   ccc tca cta ggg agg tgc ccc att att gct tca cta agt ttt ggt gcc     1039
   Pro Ser Leu Gly Arg Cys Pro Ile Ile Ala Ser Leu Ser Phe Gly Ala
                   200                 205                 210
   aca cgc aca ttt gag atg aga aag aag cca cca cca gaa gag aat gga     1087
   Thr Arg Thr Phe Glu Met Arg Lys Lys Pro Pro Pro Glu Glu Asn Gly
               215                 220                 225
   gac tac aca tat gtg gaa aga gtg aag ata ccc ttg gat cat ggg acc     1135
   Asp Tyr Thr Tyr Val Glu Arg Val Lys Ile Pro Leu Asp His Gly Thr
           230                 235                 240
   ttg tta atc atg gaa gga gcg aca caa gct gac tgg cag cat cga gtg     1183
   Leu Leu Ile Met Glu Gly Ala Thr Gln Ala Asp Trp Gln His Arg Val
       245                 250                 255
   ccc aaa gaa tac cac tct aga gaa ccg aga gtg aac ctg acc ttt cgg     1231
   Pro Lys Glu Tyr His Ser Arg Glu Pro Arg Val Asn Leu Thr Phe Arg
   260                 265                 270                 275
   aca gtc tat cca gac cct cga ggg gca ccc tgg tga cgtcagagct          1277
   Thr Val Tyr Pro Asp Pro Arg Gly Ala Pro Trp
                   280                 285
   ttgagagaga agcttcactg aaacggagca aaccttccac tgagaagcca cttcaagagg   1337
   ctggtgctgc tagatctcat gatgtggctg ttgggaagat ggtggggttt gtttgccagc   1397
   ttggagtcct attaaatgaa agccagcaac tcatgttggt aataggtcta ctgtgggaac   1457
   agttatccct aaccacagct caaaatcgct atcatcttta ggcaaattaa aatctatgtg   1517
   gca                                                                 1520
<210> 2
   <211> 286
   <212> PRT
   <213> Homo sapiens
<400> 2
   Met Glu Glu Lys Arg Arg Arg Ala Arg Val Gln Gly Ala Trp Ala Ala
   1               5                   10                  15
   Pro Val Lys Ser Gln Ala Ile Ala Gln Pro Ala Thr Thr Ala Lys Ser
               20                  25                  30
   His Leu His Gln Lys Pro Gly Gln Thr Trp Lys Asn Lys Glu His His
           35                  40                  45
   Leu Ser Asp Arg Glu Phe Val Phe Lys Glu Pro Gln Gln Val Val Arg
       50                  55                  60
   Arg Ala Pro Glu Pro Arg Val Ile Asp Arg Glu Gly Val Tyr Glu Ile
   65                  70                  75                  80
   Ser Leu Ser Pro Thr Gly Val Ser Arg Val Cys Leu Tyr Pro Gly Phe
                   85                  90                  95
   Val Asp Val Lys Glu Ala Asp Trp Ile Leu Glu Gln Leu Cys Gln Asp
               100                 105                 110
   Val Pro Trp Lys Gln Arg Thr Gly Ile Arg Glu Asp Ile Thr Tyr Gln
           115                 120                 125
   Gln Pro Arg Leu Thr Ala Trp Tyr Gly Glu Leu Pro Tyr Thr Tyr Ser
       130                 135                 140
   Arg Ile Thr Met Glu Pro Asn Pro His Trp His Pro Val Leu Arg Thr
   145                 150                 155                 160
   Leu Lys Asn Arg Ile Glu Glu Asn Thr Gly His Thr Phe Asn Ser Leu
   165 170 175
   Leu Cys Asn Leu Tyr Arg Asn Glu Lys Asp Ser Val Asp Trp His Ser
   180 185 190
   Asp Asp Glu Pro Ser Leu Gly Arg Cys Pro Ile Ile Ala Ser Leu Ser
   195 200 205
   Phe Gly Ala Thr Arg Thr Phe Glu Met Arg Lys Lys Pro Pro Pro Glu
   210 215 220
   Glu Asn Gly Asp Tyr Thr Tyr Val Glu Arg Val Lys Ile Pro Leu Asp
   225 230 235 240
   His Gly Thr Leu Leu Ile Met Glu Gly Ala Thr Gln Ala Asp Trp Gln
   245 250 255
   His Arg Val Pro Lys Glu Tyr His Ser Arg Glu Pro Arg Val Asn Leu
   260 265 270
   Thr Phe Arg Thr Val Tyr Pro Asp Pro Arg Gly Ala Pro Trp
   275 280 285
<210> 3
   <211> 22
   <212> PRT
   <213> Artificial
<220>
   <223> PCA-1 peptide fragment 1 by tryptic digestion
<400> 3
   Ile Pro Leu Asp His Gly Thr Leu Leu Ile Met Glu Gly Ala Thr Gln
   1 5 10 15
   Ala Asp Trp Gln His Arg
   20
<210> 4
   <211> 18
   <212> PRT
   <213> Artificial
<220>
   <223> PCA-1 peptide fragment 2 by tryptic digestion
<400> 4
   Ile Glu Glu Asn Thr Gly His Thr Phe Asn Ser Leu Leu Cys Asn Leu
   1 5 10 15
   Tyr Arg
<210> 5
   <211> 16
   <212> PRT
   <213> Artificial
<220>
   <223> PCA-1 peptide fragment 3 by tryptic digestion
<400> 5
   Glu Ala Asp Trp Ile Leu Glu Gln Leu Cys Gln Asp Val Pro Trp Lys
   1 5 10 15
<210> 6
   <211> 15
   <212> PRT
   <213> Artificial
<220>
   <223> PCA-1 peptide fragment 4 by tryptic digestion
<400> 6
   Lys Pro Pro Pro Glu Glu Asn Gly Asp Tyr Thr Tyr Val Glu Arg
   1 5 10 15
<210> 7
   <211> 14
   <212> PRT
   <213> Artificial
<220>
   <223> PCA-1 peptide fragment 5 by tryptic digestion
<400> 7
   Ile Thr Met Glu Pro Asn Pro His Trp His Pro Val Leu Arg
   1 5 10
<210> 8
   <211> 14
   <212> PRT
   <213> Artificial
<220>
   <223> PCA-1 peptide fragment 6 by tryptic digestion
<400> 8
   Leu Thr Ala Trp Tyr Gly Glu Leu Pro Tyr Thr Tyr Ser Arg
   1 5 10
<210> 9
   <211> 15
   <212> PRT
   <213> Artificial
<220>
   <223> PCA-1 peptide fragment 7 by tryptic digestion
<400> 9
   Asp Ser Val Asp Trp His Ser Asp Asp Glu Pro Ser Leu Gly Arg
   1 5 10 15
<210> 10
   <211> 15
   <212> PRT
   <213> Artificial
<220>
   <223> PCA-1 peptide fragment 8 by tryptic digestion
<400> 10
   Glu Gly Val Tyr Glu Ile Ser Leu Ser Pro Thr Gly Val Ser Arg
   1 5 10 15
<210> 11
   <211> 12
   <212> PRT
   <213> Artificial
<220>
   <223> PCA-1 peptide fragment 9 by tryptic digestion
<400> 11
   Ser His Leu His Gln Lys Pro Gly Gln Thr Trp Lys
   1 5 10
<210> 12
   <211> 13
   <212> PRT
   <213> Artificial
<220>
   <223> PCA-1 peptide fragment 10 by tryptic digestion
<400> 12
   Cys Pro Ile Ile Ala Ser Leu Ser Phe Gly Ala Thr Arg
   1 5 10
<210> 13
   <211> 11
   <212> PRT
   <213> Artificial
<220>
   <223> PCA-1 peptide fragment 11 by tryptic digestion
<400> 13
   Val Cys Leu Tyr Pro Gly Phe Val Asp Val Lys
   1 5 10
<210> 14
   <211> 12
   <212> PRT
   <213> Artificial
<220>
   <223> PCA-1 peptide fragment 12 by tryptic digestion
<400> 14
   Ser Gln Ala Ile Ala Gln Pro Ala Thr Thr Ala Lys
   1 5 10
<210> 15
   <211> 9
   <212> PRT
   <213> Artificial
<220>
   <223> PCA-1 peptide fragment 13 by tryptic digestion
<400> 15
   Glu Asp Ile Thr Tyr Gln Gln Pro Arg
   1 5
<210> 16
   <211> 10
   <212> PRT
   <213> Artificial
<220>
   <223> PCA-1 peptide fragment 14 by tryptic digestion
<400> 16
   Val Gln Gly Ala Trp Ala Ala Pro Val Lys
   1 5 10
<210> 17
   <211> 7
   <212> PRT
   <213> Artificial
<220>
   <223> PCA-1 peptide fragment 15 by tryptic digestion
<400> 17
   Glu His His Leu Ser Asp Arg
   1 5
<210> 18
   <211> 7
   <212> PRT
   <213> Artificial
<220>
   <223> PCA-1 peptide fragment 16 by tryptic digestion
<400> 18
   Glu Pro Gln Gln Val Val Arg
   1 5
<210> 19
   <211> 7
   <212> PRT
   <213> Artificial
<220>
   <223> PCA-1 peptide fragment 17 by tryptic digestion
<400> 19
   Thr Val Tyr Pro Asp Pro Arg
   1 5
<210> 20
   <211> 6
   <212> PRT
   <213> Artificial
<220>
   <223> PCA-1 peptide fragment 18 by tryptic digestion
<400> 20
   Val Asn Leu Thr Phe Arg
   1 5
<210> 21
   <211> 5
   <212> PRT
   <213> Artificial
<220>
   <223> PCA-1 peptide fragment 19 by tryptic digestion
<400> 21
   Glu Tyr His Ser Arg
   1 5
<210> 22
   <211> 5
   <212> PRT
   <213> Artificial
<220>
   <223> PCA-1 peptide fragment 20 by tryptic digestion
<400> 22
   Thr Phe Glu Met Arg
   1 5
<210> 23
   <211> 5
   <212> PRT
   <213> Artificial
<220>
   <223> PCA-1 peptide fragment 21 by tryptic digestion
<400> 23
   Glu Phe Val Phe Lys
   1               5
<210> 24
   <211> 37
   <212> PRT
   <213> Artificial
<220>
   <223> PSA peptide fragment 1 by tryptic digestion
<400> 24
   Pro Gln Ala Tyr His Leu His Pro Glu Ser Cys Val Thr Met Trp Val
   1               5                   10                  15
   Pro Val Val Phe Leu Thr Leu Ser Val Thr Trp Ile Gly Ala Ala Pro
               20                  25                  30
   Leu Ile Leu Ser Arg
           35
<210> 25
   <211> 30
   <212> PRT
   <213> Artificial
<220>
   <223> PSA peptide fragment 2 by tryptic digestion
<400> 25
   His Ser Leu Phe His Pro Glu Asp Thr Gly Gln Val Phe Gln Val Ser
   1               5                   10                  15
   His Ser Phe Pro His Pro Leu Tyr Asp Met Ser Leu Leu Lys
               20                  25                  30
<210> 26
   <211> 32
   <212> PRT
   <213> Artificial
<220>
   <223> PSA peptide fragment 3 by tryptic digestion
<400> 26
   Val Met Asp Leu Pro Thr Gln Glu Pro Ala Leu Gly Thr Thr Cys Tyr
   1 5 10 15
   Ala Ser Gly Trp Gly Ser Ile Glu Pro Glu Glu Phe Leu Thr Pro Lys
   20 25 30
<210> 27
   <211> 21
   <212> PRT
   <213> Artificial
<220>
   <223> PSA peptide fragment 4 by tryptic digestion
<400> 27
   Ala Val Cys Gly Gly Val Leu Val His Pro Gln Trp Val Leu Thr Ala
   1 5 10 15
   Ala His Cys Ile Arg
   20
<210> 28
   <211> 16
   <212> PRT
   <213> Artificial
<220>
   <223> PSA peptide fragment 5 by tryptic digestion
<400> 28
   Phe Leu Arg Pro Gly Asp Asp Ser Ser His Asp Leu Met Leu Leu Arg
   1 5 10 15
<210> 29
   <211> 12
   <212> PRT
   <213> Artificial
<220>
   <223> PSA peptide fragment 6 by tryptic digestion
<400> 29
   His Ser Gln Pro Trp Gln Val Leu Val Ala Ser Arg
   1 5 10
<210> 30
   <211> 12
   <212> PRT
   <213> Artificial
<220>
   <223> PSA peptide fragment 7 by tryptic digestion
<400> 30
   Leu Ser Glu Pro Ala Glu Leu Thr Asp Ala Val Lys
   1 5 10
<210> 31
   <211> 9
   <212> PRT
   <213> Artificial
<220>
   <223> PSA peptide fragment 8 by tryptic digestion
<400> 31
   Ile Val Gly Gly Trp Glu Cys Glu Lys
   1 5
<210> 32
   <211> 7
   <212> PRT
   <213> Artificial
<220>
   <223> PSA peptide fragment 9 by tryptic digestion
<400> 32
   Ser Val Ile Leu Leu Gly Arg
   1 5
<210> 33
   <211> 6
   <212> PRT
   <213> Artificial
<220>
   <223> PSA peptide fragment 10 by tryptic digestion
<400> 33
   Leu Gln Cys Val Asp Leu
   1 5
<210> 34
   <211> 13
   <212> PRT
   <213> Artificial
<220>
   <223> Peptide fragment of PCA-1
<400> 34
   Arg Arg Ala Pro Glu Pro Arg Val Ile Asp Arg Glu Gly
   1 5 10